(19) Europäisches Patentamt
European Patent Office
Office européen des brevets

(11) EP 0 759 904 B1

(12) EUROPEAN PATENT SPECIFICATION

(45) Date of publication and mention of the grant of the patent:
**05.07.2000 Bulletin 2000/27**

(21) Application number: **95916294.2**

(22) Date of filing: **10.04.1995**

(51) Int Cl.[7]: **C07D 211/22**

(86) International application number:
**PCT/US95/04422**

(87) International publication number:
**WO 95/31436 (23.11.1995 Gazette 1995/50)**

(54) **PROCESS AND DIASTEREOMERIC SALTS USEFUL FOR THE OPTICAL RESOLUTION OF RACEMIC 4-[4-[-4-(HYDROXYDIPHENYLMETHYL)-1-PIPERIDINYL]-1-HYDROXYBUTYL]-ALPHA,ALPHA-DIMETHYLBENZENE ACETIC ACID.**

VERFAHREN UND DIASTEREOMERE SALZE NÜTZLICH BEI DER RACEMATTRENNUNG VON RACEMISCHER 4-[4-[4-(HYDROXYDIPHENYLMETHYL)-1-PIPERIDINYL]-1-HYDROXYBUTYL]-ALPHA,ALPHA-DI METHYLPHENYL ESSIGSÄURE SOWIE ENTSPRECHENDE DIASTEREOMERE SALZE

PROCEDE ET SELS DIASTEREOMERES UTILES POUR LA RESOLUTION OPTIQUE DE L'ACIDE 4-[4-[4-(HYDROXYDIPHENYLMETHYL)-1-PIPERIDINYL]-1-HYDROXYBUTYL]-ALPHA,ALPHA-DI METHYLBENZENE ACETIQUE RACEMIQUE

(84) Designated Contracting States:
**AT BE CH DE DK ES FR GB GR IE IT LI LU MC NL PT SE**
Designated Extension States:
**LT SI**

(30) Priority: **16.05.1994 US 242919**

(43) Date of publication of application:
**05.03.1997 Bulletin 1997/10**

(73) Proprietor: **MERRELL PHARMACEUTICALS INC.**
**Cincinnati, Ohio 45215-6300 (US)**

(72) Inventors:
- **NAKAMURA, Mitsuo**
**Yamashina-ku, Kyoto 607 (JP)**
- **SHIGA, Masatoshi**
**Sohraku, Kyoto 619-11 (JP)**

(74) Representative: **VOSSIUS & PARTNER**
**Siebertstrasse 4**
**81675 München (DE)**

(56) References cited:
**US-A- 3 878 217**

- **PHARM. WORLD SCI., vol.15, no.5, 1993 pages 186 - 192 ZHANG M. Q., ZIMMERMAN H. 'Terfenadine: a mixture of equipotent antihistamine enantiomers without a clear 'isomeric ballast'.'**
- **JACQUES J., COLLET A., WILEN S. H. 'Enantiomers, Racemates and Resolutions' 1991 , KRIEGER PUBLISHING COMPANY , MALABAR, FLORIDA page 259 - 261, formulae 25, 27 and 40**
- **A. Terhechte und G. Blashke, "Indirekte und direkte Racematrennung von Terfenadine und seines Hauptmetaboliten, MDL 16,455A", Arch. Pharm.,1991, vol. 324, page 710**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 759 904 B1

## Description

This invention relates to the resolution of racemic compositions, more particularly to a process for resolving racemic 4-[4-[4-(hydroxydiphenylmethyl)-1-piperidinyl]-1-hydroxybutyl]-α,α-dimethylbenzeneacetic acid.

There are presently many methods available for the resolution of racemic compounds. For example, familiar techniques include formation of diastereomers followed by crystallization, differential absorption (chromatography), biochemical processes, chiral recognition, direct crystallization, differential reactivity and mechanical separation. Industrial scale resolution of optical isomers requires that both efficiency and economy of any resolving technique be high in order for such procedure to be practical, and thus feasible.

The method of optical resolution incorporating the formation of a diastereomeric complex with a chiral resolving agent and a single enantiomer of the racemic compound and subsequent crystallization of the complex has been traditionally a very significant technique of optical resolution. Also known as fractional crystallization, it is very tedious in that the choice of suitable solvents and chiral resolving agents is largely a matter of trial and error. The technique is further limited in that it is only applicable to solids. As a result, a search for other methods of efficient optical resolution is ongoing. As a result, the recognition of fractional crystallization as an important optical resolution tool and potential for commercial exploitation has been diminishing in recent years.

Numerous chiral resolving agents have been available and are known. However, as mentioned previously, useful chiral resolving agents for crystallization on an industrial scale have particular requirements. For example, they should be relatively inexpensive and of a high state of optical purity. They should react easily with the desired target enantiomer and form a diastereomeric complex with physical properties sufficiently different from other associative complexes in the solution so as to precipitate relatively exclusively, and in a state free from the other associative complexes. Precipitation in such degree of relative exclusivity is necessary in order to achieve a high degree of optical purity of the enantiomeric target compound. Additionally, good resolving agents should be recyclable, that is, recoverable from the solution in significant quantitative yield. These additional practical restraints have made the use of chiral resolving agents for resolution on an industrial scale even less of a viable tool.

The compound α-[4-(1,1-dimethylethyl)phenyl]-4-(hydroxydiphenylmethyl)-1-piperidinebutanol, more commonly known as terfenadine and various of its derivatives are known to have great utility as antihistamines, antiallergy agents, and bronchodilators, as is described in U.S. Pat. No. 3,878,217 to Carr et al. (Carr I) and U.S. Pat. No. 4,254,129 to Carr et al. (Carr II).

Despite the difficulties in the discovery of suitable resolving agents having utility for optical resolutions on an industrial scale, one chiral resolving agent has been previously used for the optical resolution of terfenadine. Carr I discloses a process for resolving both the dextro and levo rotatory isomers of terfenadine using (-)-binaphthylphosphoric acid and (+)-binaphthylphosphoric acid, also known as (-)/(+)-1,1'-binaphthyl-2,2'-diyl hydrogen phosphate.

M. Q. Zhang, et al., Pharm. World Sci., Vol. 15, No. 5, pages 186-192 (1993) describes different methods and approaches to obtain pure enantiomers of terfenadine and to the *in vitro* antihistamine activity, central nervous system side-effects, metabolism and calcium channel affinity of terfenadine enantiomers.

A. Terhecte, et al., Arch. Pharm. 1991:324:710 describes the indirect separation of terfenadine by preparative diastereoisomeric separation and a direct separation of terfenadine and the chiral main metabolite, MDL 16,455A by chiral phases Chiralcel-OD and Chiralpak-AD, as well as on Ultron-ES-OVM, a protein phase.

An object of this invention is to provide an improved process for the optical resolution of racemic 4-[4-[4-(hydroxydiphenylmethyl)-1-piperidinyl]-1-hydcoxybutyl]-α,α-dimethylbenzeneacetic acid.

A further object of this invention is to provide a resolving process which is both efficient and economical. Reaction schemes A and B graphically illustrate the process incorporating di-para-toluoyltartaric acid and mandelic acid, respectively to complete a separation scheme for the (R) and (S) enantiomers of terfenadine, of the acid derivative and of the acid ester derivative compounds of the invention. Unless otherwise noted in reaction schemes A & B, the appearance of two signs in parentheses refers to a diastereomeric salt wherein the first sign refers to the target molecule and the second sign denotes the resolving agent.

**SCHEME A**

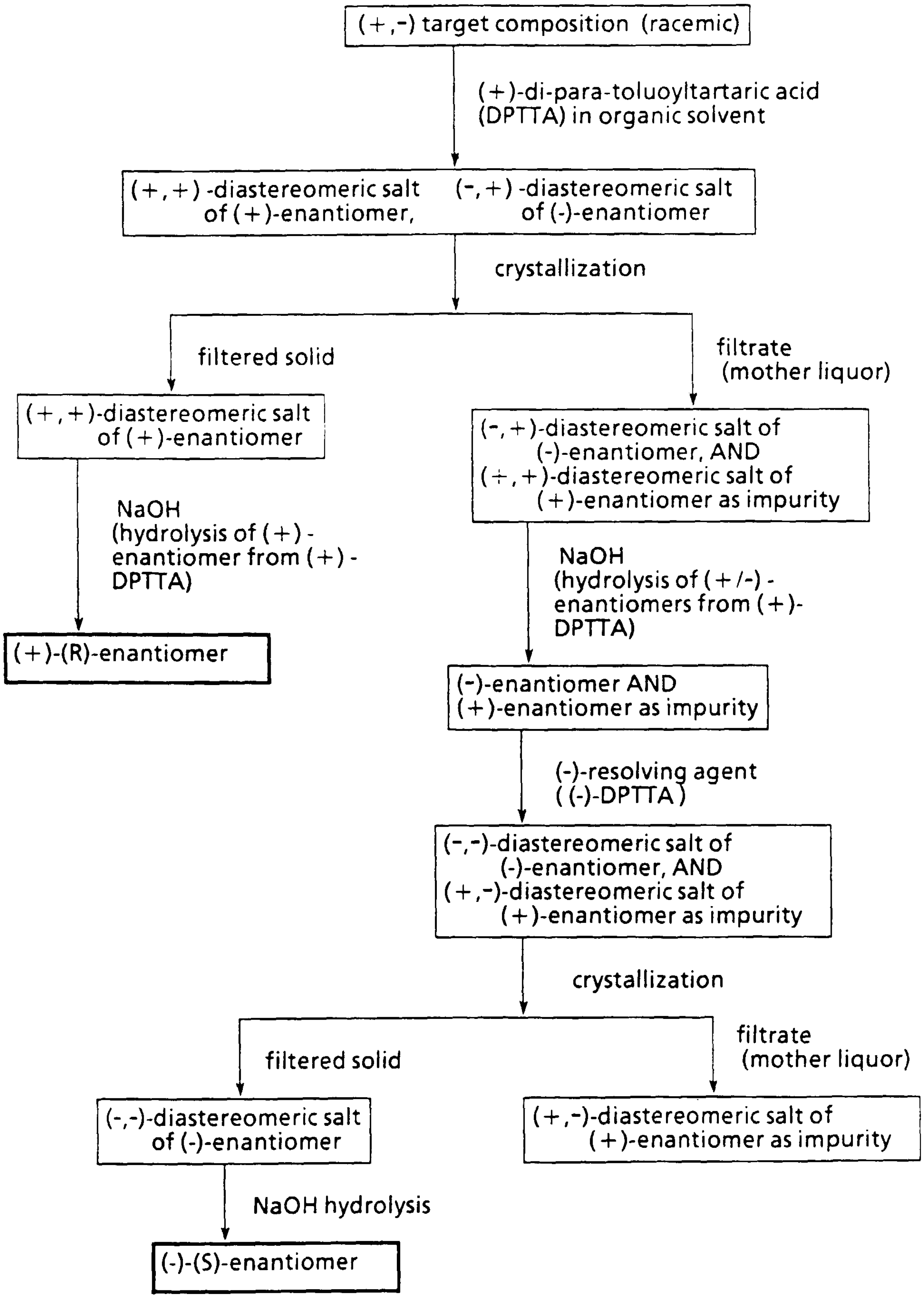
(+,-) target composition (racemic)
(+)-di-para-toluoyltartaric acid (DPTTA) in organic solvent
(+,+) -diastereomeric salt of (+)-enantiomer,
(-,+) -diastereomeric salt of (-)-enantiomer
crystallization
filtered solid
filtrate (mother liquor)
(+,+)-diastereomeric salt of (+)-enantiomer
(-,+)-diastereomeric salt of (-)-enantiomer, AND (+,+)-diastereomeric salt of (+)-enantiomer as impurity
NaOH (hydrolysis of (+)-enantiomer from (+)-DPTTA)
NaOH (hydrolysis of (+/-)-enantiomers from (+)-DPTTA)
(+)-(R)-enantiomer
(-)-enantiomer AND (+)-enantiomer as impurity
(-)-resolving agent ((-)-DPTTA)
(-,-)-diastereomeric salt of (-)-enantiomer, AND (+,-)-diastereomeric salt of (+)-enantiomer as impurity
crystallization
filtered solid
filtrate (mother liquor)
(-,-)-diastereomeric salt of (-)-enantiomer
(+,-)-diastereomeric salt of (+)-enantiomer as impurity
NaOH hydrolysis
(-)-(S)-enantiomer

SCHEME B

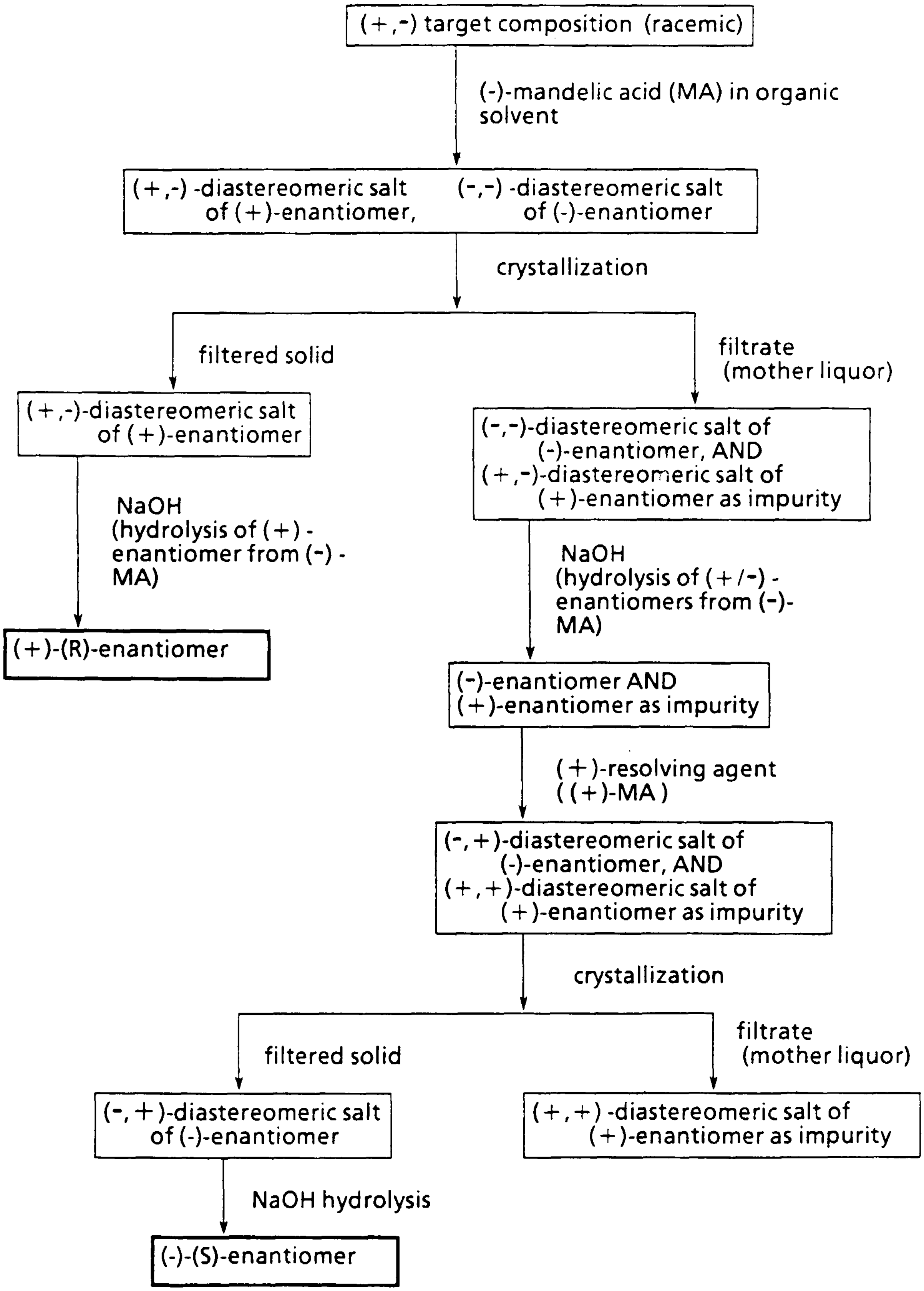


These objects and more are fulfilled by the process of preparing compounds of the formula:

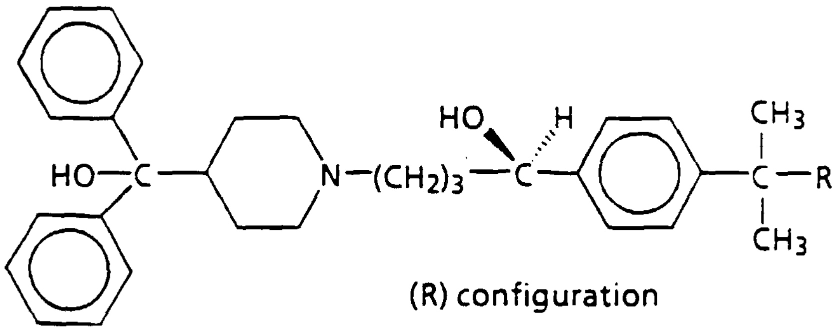

(R) configuration

II

wherein R is -COOH; the notation;

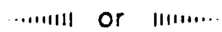

or

indicates a bond which protrudes back from the plane of the paper;
the notation:

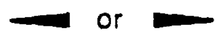

or

indicates a bond which protrudes forward from the plane of the paper; and
the notation:



indicates a bond for which the stereochemistry is not designated (a racemic composition);
comprising:

a) dissolving into a solution an amount of a racemic compound of a formula:

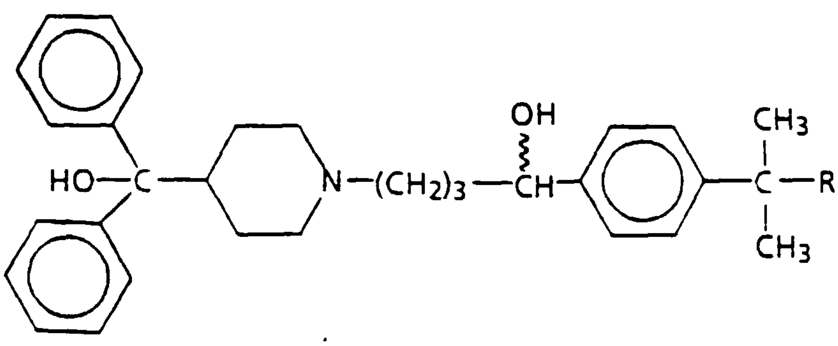

I

wherein R and the bond notations are defined as above; with an equimolar amount of optically active resolving agent, (+)-di-para-toluoyltartaric acid, into a suitable organic solvent;
b) heating the solution to an elevated temperature suitable for formation of a solubilized diastereomeric salt between the optically active resolving agent and the compound;
c) cooling the solution for a period of time sufficient to precipitate the diastereomeric salt;
d) collecting the diastereomeric salt; and
e) hydrolysing the diastereomeric salt to isolate the compound.

Similarly, the following process can prepare compounds of a formula:

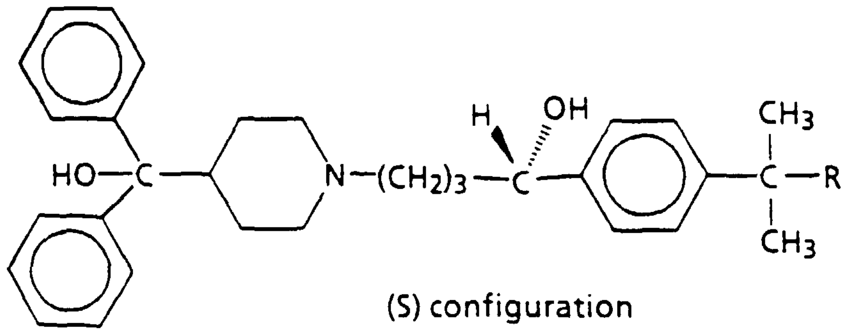

III

(S) configuration

wherein R is -COOH and the bond notations are defined as above;
comprising:

a) dissolving into a solution an amount of a racemic compound of a formula:

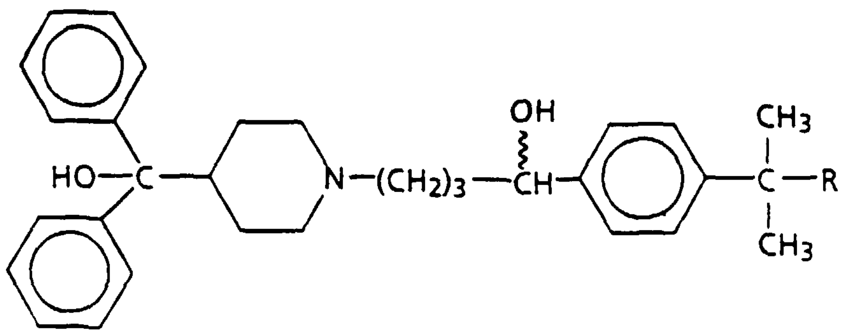

I

wherein R and the bond notations are defined as above; with an equimolar amount of an optically active resolving agent, (+)-di-para-toluoyltartaric acid, into a suitable organic solvent;
b) heating the solution to an elevated temperature suitable for formation of a first solubilized diastereomeric salt between the optically active resolving agent and the compound;
c) cooling the solution for a period of time sufficient to precipitate the first diastereomeric salt;
d) removing the first diastereomeric salt and preserving the solution as a filtrate;
e) hydrolysing and separating the compound from the filtrate;
f) dissolving into solution the compound with an optically active resolving agent', (-)-di-para-toluoyltartaric acid in an amount equimolar to an amount of the compound in such manner as to form a second solubilized diastereomeric salt between the same;
g) precipitating the second diastereomeric salt;
h) collecting the second diastereomeric salt; and
i) hydrolysing the second diastereomeric salt to isolate the compound.

It should further be appreciated that while reaction Schemes A and B as well as the above description detail a process whereby the (R) enantiomer is crystallized first from the solution by association with the chiral resolving agent, while the (S) enantiomer remains in solution for subsequent crystallization with the resolving agent', the order of the crystallization can be reversed. That is, the (S) enantiomer may be crystallized by association with the resolving agent' first while the (R) enantiomer remains in solution and may be isolated subsequently by association with the resolving agent.

It is a still further object of the invention to provide diastereomeric salts useful for the resolution of racemic 4-[4-[4-(hydroxydiphenylmethyl)-1-piperidinyl]-1-hydroxybutyl]-$\alpha$,$\alpha$-dimethylbenzeneacetic acid.

As used herein, "lower alkyl ester" refers to a compound wherein the R group of compounds I, II or III has been substituted with a carboxylic acid ester functional moiety of from one to five carbon atoms. For example, methoxycarbonyl, ethoxycarbonyl, n-propoxycarbonyl, isopropoxycarbonyl, n-butoxycarbonyl, isobutoxycarbonyl, t-butoxycarbonyl and the like.

As used herein, "chiral resolving agent" or "optically active resolving agent" refers to either the dextro or levo

rotatory optical isomer of the following compounds: di-para-toluoyltartaric acid and mandelic acid. "Resolving agent" and "resolving agent'" designate enantiomers of the same compound.

As used herein, the term "suitable organic solvent" refers to any polar organic solvent in which the interactive complex formed between the chiral resolving agent and the piperidinebutanol is soluble at an elevated temperature but insoluble at ambient temperatures. Suitable organic solvents may also be employed during the recrystallization of the target enantiomeric compound. For example, there may be mentioned methanol, ethanol and acetone.

The "elevated temperature" facilitating formation of the interactive complex may be any temperature at which the complex is soluble, but is typically in the range of about 50°C to about 100°C. When the organic solvent is acetone the range is about 50°C to about 55°C.

As used herein the term "salt" or "diastereomeric salt" has the general meaning imputed to the term by the art. For example, it can refer to the associative complex which results when the anionic element of an acidic chiral resolving agent associates with the cationic portion of the desired enantiomer of a basic racemic target compound (enantiomer) which results from one or more points of interaction due to one or more weak attractive forces. The term "solubilized diastereomeric salt" refers to a diastereomeric salt formed in solution. A solubilized diastereomeric salt can exhibit physical properties different from other associative complexes in the solution. These physical differences, (e.g. association equilibria, crystallization energies, etc.) can be exploited so that the diastereomeric salt formed between the target enantiomer and the chiral resolving agent precipitates while the other associative complexes (chiral resolving agent with enantiomer of target, impurities, double salt-complexes, etc.) remain in solution. The magnitude and extent of the differential in the attractive forces between the chiral resolving agent and each enantiomer of the racemic target composition, which in turn control the precipitation of the desired salt, may also be affected by the choice of organic solvent.

The temperature to which the solution is cooled can be any temperature lower than the temperature at which the interactive complex begins to precipitate, but is typically between -20°C and 40°C. Preferably, it is -10°C to 30°C and most preferably it is 4°C to 25°C.

The period of time for which the solution is cooled is a time period sufficient for the diastereomeric salt in the solution to precipitate. It can vary depending upon temperature and degree of agitation during the crystallization period, but is typically between 0.5 day and 10 days. Preferably it is between 0.5 day and 3 days, and most preferably it is between 1 day and 2 days.

The following examples are given to illustrate in more intricate detail, but they should not be construed as limiting the invention in any way.

Except where otherwise noted, the physical analyses were conducted on the following equipment: Hot stage melting points were determined on a YANAGIMOTO® micro melting point apparatus (Model MP) and are uncorrected, while capillary melting points were determined on a YAMATO® melting point apparatus (Model MP-21), and are also uncorrected values; NMR spectra were taken on a HITACHI® R-90H Fourier transform NMR spectrometer with chemical shifts reported, unless otherwise noted, in δ units relative to internal tetramethylsilane; IR spectra were measured with a HITACHI® 260-10 infrared spectrophotometer. Specific rotations were measured with a JASCO® DIP-370 digital polarimeter. HPLC was taken on a WATERS® liquid chromatograph consisting of a model 510 pump, U6K injector and 990J photodiode array detector. Chemical yield of the diastereomeric salts (interactive complexes) and the enantiomers were calculated based on half the amount of the racemic compound used.

In the examples following, the optical purity was determined by chiral HPLC. Unless indicated otherwise, the analysis for terfenadine (both (+) and (-) enantiomers) incorporated the following parameters:

| | |
|---|---|
| Column: | Size, 4.6 X 150 mm Stationary phase, ULTRON®ES-OVM (5µm) (SHINWA CHEMICAL INDUSTRIES) |
| Wavelength: | 210 nm |
| Mobile Phase: | $CH_3CN$-0.05M sodium phosphate buffer (pH 6.0) (20:80) |
| Flow Rate: | 1.0 ml/min. |
| Sample: | 5µl (0.05% solution in methanol) |

Unless otherwise indicated, before running HPLC analysis the ethyl 4-α,α-dimethylbenzeneacetate derivative was converted into the 4-α,α-dimethylbenzeneacetic acid derivative. The analysis of the acid incorporated the following parameters:

| | |
|---|---|
| Column: | Size, 4.6 X 150 mm Stationary phase, ULTRON®ES-OVM (5µm) (SHINWA CHEMICAL INDUS- |

TRIES)

Wavelength: 210 nm

Mobile Phase: $CH_3CN$-0.05M sodium phosphate buffer (pH 4.5) (6:94)

Flow Rate: 1.0 ml/min.

Sample: The sample (ca. 5 mg) was dissolved in EtOH (2 ml) and then 2N-NaOH (1 ml) was added. The solution was transferred into an ampule. The ampule was sealed by melting an end in fire and was placed in a waterbath set at 80°C for 2 hr. After neutralization with 2N HCl (1ml), the solution was diluted with EtOH to 10 ml. The solution (5 μl) was injected for analysis.

Resolution of terfenadine

Reference Example 1A

(R)-(+)-terfenadine

Racemic α-[4-(1,1-dimethylethyl)phenyl]-4-(hydroxy-diphenylmethyl)-1-piperidinebutanol (terfenadine)(10.0 g, 21.2 mmole) and (2S,3S)-(+)-di-para-toluoyltartaric acid monohydrate ((+)-DPTTA)(8.60 g, 21.3 mmole) were dissolved in 90 ml acetone by heating to ca. 55°C. The resulting solution was cooled at room temperature (15° to 30°C) for one day and then in a refrigerator for an additional day. The resulting crystals were collected by filtration yielding a precipitated diastereomeric salt comprising (+)-terfenadine and (+)-DPTTA (98% chemical yield, 90% diastereomeric excess (%de)).

The salt was recrystallized twice from ca. 8 ml acetone per gram of salt and dried at 80°C in vacuo for one day to give a purified diastereomeric salt (7.54 g, 83% chemical yield, ca. 100 %de). mp. ca. 125-134°C (hot stage)
IR (KBr): 2800-2200, 1720, 1610, 1265, 1105 $cm^{-1}$.

$[\alpha]_D^{24}$ +20° (c=1.0,*$CHCl_3$*)

Analysis calculated for $C_{52}H_{59}NO_{10}\cdot(0.5)H_2O$: C:72.03; H:6.97; N:1.62; Found: C:72.11; H:6.99; N:1.60.

The diastereomeric salt (7.04 g) was then dissolved into 45 ml of ethanol. To this solution was added 16.5 ml of 1N NaOH and then 30 ml $H_2O$. The resulting crystals were collected and recrystallized once from ethanol/$H_2O$ (1:1) to give optically pure (ca. 100 %ee) (R)-(+)-terfenadine (3.81 g, chemical yield of 81%). mp. 145-146°C.

$[\alpha]_D^{24}$ +50° (*c*=4.0,*$CHCl_3$*)

$^1$H-NMR [$CDCl_3$] δ; 7.1-7.6 (14H, m, aromatic H), 4.5-4.7 (1H, m., <u>CH</u>-OH), ca. 3.05 (2H, bd.trip, J=13Hz, axial H of N-$CH_2$ x2 in piperidine ring), 1.4-2.5 (14H, m., remaining H), 2.25 (1H, s., OH), 1.29 (9H, s, t-but.-H).
Analysis calculated for $C_{32}H_{41}NO_2$: C:81.49; H:8.76; N:2.97. Found: C: 81.43; H: 8.72; N: 2.84.

The experimental results and certain parameters from the crystallization are graphically illustrated in Table 1, where a comparison may be made with other resolving agents and organic solvents.

Reference EXAMPLE 1B

(S)-(-)-terfenadine

To the mother liquor from the crystallization of the diastereomeric salt of (R)-(+)-terfenadine and (2S,3S)-(+) di-para-toluoyltartaric acid was added 22 ml of 1N NaOH and then 80 ml of $H_2O$. The resulting crystals were collected and recrystallized once from ethanol/$H_2O$ yielding partially resolved (S)-(-)-terfenadine in 96% chemical yield (4.81 g).

The crystals were then combined with an equimolar proportion of (2R,3R)-(-)-di-para-toluoyltartaric acid (3.94 g, 10.2 mmole) in 75 ml of acetone and remained at room temperature (15°C to 30°C) for one day and then in a refrigerator for an additional day. The resulting crystals were collected by filtration to yield the diastereomeric salt of (S)-(-)-terfenadine and (-)-di-para-toluoyltartaric acid. The salt was recrystallized once from ca. 8 ml acetone per gram of salt and dried at 80°C in vacuo for one day to give purified diastereomeric crystals (7.03 g, 77% chemical yield) with an optical purity of ca. 100% diastereomeric excess. mp. ca. 125-134°C (hot stage).
IR (KBr): 2800-2200, 1720, 1610, 1265, 1105 $cm^{-1}$

$[\alpha]_D^{24}$ -21° (*c*=1.0,*$CHCl_3$*)

Analysis calculated for $C_{52}H_{59}NO_{10}\cdot(0.5)H_2O$: C:72.03; H:6.97; N:1.62. Found: C:72.10; H:6.95; N:1.62.

The diastereomeric crystals (6.53 g) were then dissolved into 45 ml ethanol to which was added 15.5 ml of 1N NaOH and then 30 ml $H_2O$. The resulting crystals were collected and recrystallized once from ethanol/$H_2O$ (1:1)

to give (S)-(-)-terfenadine (3.53 g, 75% chemical yield) having an optical purity of ca. 100% enantiomeric excess. mp. 145-146°C.
$^1$H-NMR ($CDCl_3$), δ; 7.1-7.6 (14H, m. aromatic H), 4.5-4.7 (1H, m. CH-OH), ca. 3.05 (2H, bd.trip., J=13Hz, axial H of N-$CH_2$ x2 in the piperidine ring), 1.4-2.5 (14H, m., remaining H), 2.25 (1H, s., -OH), 1.29 (9H, s., t-butyl-H)

$[\alpha]_D^{24}$ -50° (*c*=4.0,*CHCl*$_3$)

Analysis calculated for $C_{32}H_{41}NO_2$: C:81.49; H:8.76; N:2.97. Found: C:81.48; H:8.74; N:2.84.

Reference Example 2A

(R)-(+)-terfenadine

Racemic terfenadine (20 g, 42.4 mmole) and (R)-(-)-mandelic acid (6.45 g, 42.4 mmole) were dissolved in 180 ml of methanol by heating to ca. 60°C. The resulting solution was cooled to room temperature (15°C to 30°C) for 1 day and in a refrigerator set to 4°C for another day. The resulting crystals were collected by filtration over a vacuum to give the crystalline diastereomeric salt comprising the resolving agent and the (+)-enantiomer (101% chem. yield, 78%de). The crystals were then recrystallized twice from ca. 9 ml methanol per gram of salt and dried at 80°C in vacuo for one day to yield purified diastereomeric crystals (9.70 g, 73% chemical yield, 99%de). m.p. ca. 112-118°C (hot stage) IR (KBr): 2800-2100, 1610, 1360 $cm^{-1}$.

$[\alpha]_D^{23}$ -5.9° (*c*=2.0, *CHCl*$_3$)

Analysis calculated for $C_{40}H_{49}NO_5$: C:77.01; H:7.92; N:2.25. Found: C:77.14; H:8.03; N:2.29.

The purified diastereomeric crystals (9.10 g) were dissolved in 60 ml ethanol. To this solution was added 15.0 ml of 1N NaOH and 45 ml of $H_2O$. The resulting crystals were then collected and recrystallized once from ethanol/$E_2O$ (1:1) to yield the (R)-(+)-enantiomer (6.40 g, 68% chemical yield) with an optical purity of 99% enantiomeric excess. m.p. 145-146°C.

$[\alpha]_D^{23}$ +51° (*c*=4.0,*CHCl*$_3$)

Analysis calculated for $C_{32}H_{41}NO_2$: C:81.49; H:8.76; N:2.97.
Found: C: 81.68; H:8.81; N:2.85.

The crystallization of (R)-(+)-terfenadine with (R)-(-)-mandelic acid and certain experimental parameters are graphically illustrated in Table 1. Table 1 permits a comparison in the feasibility and efficiency between various resolving agents and organic solvents.

Reference Example 2B

(S)-(-)-terfenadine

To the mother liquor from the crystallization of (R)-(+)-terfenadine and (R)-(-)-mandelic acid was added 23 ml of 1N NaOH and then 150 ml of $H_2O$. The resulting crystals were collected and recrystallized once from ethanol/$H_2O$ (1:1) to give partially resolved (S)-(-)-terfenadine (9.80 g, 98% chemical yield). The crude crystals were combined with an equimolar proportion of (S)-(+)-mandelic acid (20.8 mmole, 3.16 g) in 120 ml of methanol and remained at room temperature (15°C to 30°C) for one day and then in a refrigerator set to 4°C for another day. The crystals were collected by filtration to give a crude diastereomeric salt product of (S)-(-)-terfenadine and (S)-(+)-mandelic acid. This crude salt was recrystallized once from ca. 9 ml methanol per gram of salt and dried at 80°C in vacuo for one day to give purified diastereomeric salt in 76% chemical yield (10.0 g, 98%de). mp. ca. 112-119°C (hot stage).
IR (KBr): 2800-2100, 1610, 1360 $cm^{-1}$

$[\alpha]_D^{23}$ +5.5° (*c*=2.0, *CHCl*$_3$)

Analysis calculated for $C_{40}H_{49}NO_5$: C:77.01; H:7.92; N:2.25.
Found C:76.75; H:8.04; N:2.22.

The purified salt (9.5 g) was dissolved into 60 ml of ethanol and then treated with 15.5 ml 1N NaOH, followed by 45 ml $H_2O$. The resulting crystals were collected and recrystallized once from ethanol/$H_2O$ (1:1) to give optically pure (S)-(-)-terfenadine (6.61 g, 70% chemical yield). mp. 144-145°C.

$[\alpha]_D^{23}$ -49° (*c*=4.0,*CHCl*$_3$)

The optical purity was determined to be 98% enantiomeric excess. Analysis calculated for $C_{32}H_{41}NO_2$: C:81.49; H: 8.76; N:2.97. Found C:81.47; H:8.76; N:2.94.

Comparative Example 1

(R)-(+)-terfenadine

Following the method of optical resolution disclosed in U.S. Patent 3,878,217, racemic terfenadine α-[4-(1,1-dimethylethyl)phenyl]-4-(hydroxydiphenylmethyl)-1-piperidinebutanol, 40.8 g, 86.5 mmol) and (R)-(-)-1,1'-binaphthyl-2,2'-diyl hydrogen phosphate (30.0 g, 86.1 mmol) were mixed into 250 ml of methanol and heated to near refluxing temperature to form a solution. The solution was cooled to room temperature (15°C to 30°C) for 5 hours. The reaction vessel was then cooled to 5°C for 20 hours, after which the crystals were collected. The crystals were then recrystallized seven times from methanol by dissolving in 3-7 ml per gram of the crystals to be placed into solution and the final crystallization was cooled to 5°C overnight (15-20 hours) to give the crystalline diastereomeric salt comprising of (R)-(-)-1,1'-binaphthyl-2,2'-diyl hydrogen phosphate and (R)-(+)-α-[4-(1,1-dimethylethyl)phenyl]-4-(hydroxydiphenylmethyl)-1-piperidinebutanol (8.5 g, 24% chem. yield).

The salt was dissolved in 80 ml of acetone, treated with 8 ml of aq. 10% sodium hydroxide solution, and water was added until the solution became turbid. The solution was cooled at room temperature (15°C to 30°C) overnight (ca. 20 hours) and filtered. The solid was recrystallized twice by dissolving in 80 ml warm acetone and adding water until the solution became turbid to give the title compound (4.28 g.), mp 145-146°C, in 21.0% chemical yield.

$[\alpha]_D^{26}$ +49° (*c*=4.10, $CHCl_3$)

Analysis calculated for $C_{32}H_{41}NO_2$: C:81.49; H:8.76; N:2.97.

Found: C:81.40; H:8.92; N:2.99. The enantiomeric purity was 98 % enantiomeric excess by the method of chiral HPLC with the following parameters:

| | |
|---|---|
| Column : | size, 4.6 x 150 mm stationary phase, ULTRON® ES-OVM(5μm) (SHINWA CHEMICAL INDUSTRIES, LTD.) |
| Wavelength : | 210 nm |
| Mobile phase: | $CH_3CN$-0.05M sodium phosphate buffer (pH 6.0) (20/80) |
| Flow rate : | 1.0 ml/min. |
| Sample : | 10 μl (0.02% solution in methanol) |

Reference Example 3

(R)-(+)-terfenadine

Racemic α-[4-(1,1-dimethylethyl)phenyl]-4-(hydroxy-diphenylmethyl)-1-piperidinebutanol (500 mg, 1.1 mmole) and equimolar amounts of the resolving agent were dissolved together in the organic solvent by heating to almost reflux temperature. Once the solutes completely went into solution, the reaction vessel was cooled to room temperature (15°C to 30°C) for 3 to 8 days in an environment free of disturbances in order to crystallize the diastereomeric salt. The crystals were dried over a vacuum source.

Table 1 recites a comparison between Reference Examples 1A, 2A, 3A-M and the comparative example and illustrates the result of various combinations of resolving agents and organic solvents.

From a comparison between Reference Examples 1A, 2A and 3A-M with the comparative example in Table 1, it is readily apparent that the use of the resolving agents (+)-di-para-toluoyltartaric acid and (R)-(-)-mandelic acid give greater chemical yields, are less procedurally cumbersome (2 recrystallizations as opposed to seven) and result in greater optical purity of the (+)-terfenadine enantiomer than does the use of (-)-1,1'-binaphthyl-2,2'-diyl hydrogen phosphate.

Table 1:

| Optical Resolution of Terfenadine with a Variety of Resolving Agents in Various Solvents | | | | | | | |
|---|---|---|---|---|---|---|---|
| Reference Example | Resolving Agent | Organic solvent | Diastereomer formed | Reaction yield (%)[a] | | Optical Purity (% de, ee)[b] | |
| | | | | 1 cryst | (x) recryst | 1 cryst | (x) recryst |
| 1A | (+)-DPTTA $\cdot H_2O$ | acetone | (+)-isomer/ (+)-DPTTA | 98 | (1x) 81 | 90 | (1x) 100 |
| 2A | (-)-M.A. | methanol | (+)-isomer/ (-)-M.A. | 101 | (1x) 68 | 78 | (1x) 99 |
| 3A | abietic acid | ethanol | none | -- | -- | -- | -- |
| 3B | (+)-camphoric acid | ethanol | none | -- | -- | -- | -- |
| 3C | (-)-camphorsulphonic acid | ethanol | none | -- | -- | -- | -- |
| 3D | (+)-DPTTA $\cdot H_2O$ | ethanol | (+)-isomer/ (+)-DPTTA | 96 | -- | 24 | -- |
| 3E | L-malic acid | ethanol | none | -- | -- | -- | -- |
| 3F | (-)-M.A. | ethanol | (+)-isomer/ (-)-M.A. | 93 | -- | 74 | -- |
| 3G | (-)-M.A. | acetone | none | -- | -- | -- | -- |
| 3H | (-)-M.A. | $CH_2CHOEt$ | none | -- | -- | -- | -- |
| 3I | (-)-M.A. | 2-butanone | none | -- | -- | -- | -- |
| 3J | (-)-M.A. | $CH_3CN$ | none | -- | -- | -- | -- |
| 3K | (-)-M.A. | dioxane | none | -- | -- | -- | -- |
| 3L | L-PCA | ethanol | none | -- | -- | -- | -- |
| 3M | L-tartaric acid | ethanol | none | -- | -- | -- | -- |
| Comp. 1[c] | (-)-BNDHP | methanol | (+)-isomer/ (-)-BNDHP | 102 | (2x) 21 | 18 | (2x) 98 |

KEY

DPTTA = di-para-toluoyltartaric acid

M.A. = mandelic acid

L-PCA = L-2-pyrrolidone-5-carboxylic acid

BNDHP = 1,1'-binaphthyl-2,2'-diyl hydrogen phosphate

[a]First column gives % reaction yield of diastereomeric salt based on half the amount of the racemic compound used. Second column reflects reaction yield after (x) recrystallizations of enantiomer after initial separation.

[b]Optical purity measured by chiral HPLC analysis. First column gives % optical purity in diastereomeric excess after initial crystallization of diastereomeric complex. Second column gives optical purity in enantiomeric excess after (x) recrystallizations of separated enantiomer.

[c]Comparative example uses procedure of optical resolution given in U.S.P. 3,878,217.

Table 2:

| Experimental Conditions for the Resolution of Terfenadine | | | | | | |
|---|---|---|---|---|---|---|
| Reference Example | Resolving Agent | | Organic solvent | | Reaction Conditions | |
| | type | (mg) | type | ml | Temp.[a] | Time (days) |
| 3A | abietic acid | 320 | ethanol | 2 | r.t. | 3 |
| 3B | (+)-camphoric acid | 212 | ethanol | 2 | r.t. | 3 |
| 3C | (-)-camphorsulphonic acid | 246 | ethanol | 2 | r.t. | 3 |
| 3D | (+)-DPTTA $\cdot H_2O$ | 430 | ethanol | 3 | r.t. | 8 |
| 3E | L-malic acid | 142 | ethanol | 2 | r.t. | 3 |
| 3F | (-)-M.A. | 170 | ethanol | 8 | r.t. | 6 |
| 3G | (-)-M.A. | 170 | acetone | 2 | r.t. | 8 |
| 3H | (-)-M.A. | 170 | ethyl acetate | 2 | r.t. | 8 |
| 3I | (-)-M.A. | 170 | 2-butanone | 2 | r.t. | 8 |
| 3J | (-)-M.A. | 170 | $CH_3CN$ | 2 | r.t. | 8 |
| 3K | (-)-M.A. | 170 | dioxane | 2 | r.t. | 8 |
| 3L | L-PCA | 136 | ethanol | 2 | r.t. | 3 |
| 3M | L-tartaric acid | 160 | ethanol | 3 | r.t. | 3 |

**KEY**

DPTTA = di-para-toluoyltartaric acid
M.A. = mandelic acid
L-PCA = L-2-pyrrolidone-5-carboxylic acid
BNDHP = 1,1'-binaphthyl-2,2'-diyl hydrogen phosphate
[a]r.t. = room temperature = 15°C to 30°C

Resolution of 4-α,α-dimethylbenzeneacetic acid derivative

In the following Examples 1A and 1B, NMR spectra were taken on a HITACHI® R-1900 Fourier transform NMR spectrometer, and the parameters of the assay determining optical purity were:

| | |
|---|---|
| Column: | Size, 4.6 x 150 mm Stationary phase, ULTRON®ES-OVM (5μm) SHINWA CHEMICAL INDUSTRIES |
| Wavelength: | 210 nm |
| Mobil phase: | $CH_3CN$-0.05M sodium phosphate buffer (pH 4.5)(6:94) |
| Flow rate: | 1.0 ml/min. |
| Sample: | 5-7 μl (0.05% solution in methanol) |

Example 1A

(R)-(+)-4-[4-[4-(hydroxydiphenylmethyl)-1-piperidinyl]-1-hydroxybutyl]-α,α-dimethylbenzeneacetic acid

Well dried racemic 4-[4-[4-(hydroxydiphenylmethyl)-1-piperidinyl]-1-hydroxybutyl]-α,α-dimethylbenzeneacetic acid (8.00 g., 15.9 mmole) and (+)-di-para-toluoyltartaric acid monohydrate (6.45 g, 16.0 mmole) were dissolved together in 50 ml of acetone by heating at ca. 55°C. After cooling in a refrigerator set to 4°C for 3 days, the precipitated crystals were collected by filtration to yield the diastereomeric salt comprising (+)-4-[4-[4-(hydroxydiphenylmethyl)-1-piperidinyl]-1-hydroxybutyl]-α,α-dimethylbenzeneacetic acid associated with (2S,3S)-(+)-di-para-toluoyltartaric acid (7.53 g, 107% chemical yield, 74%de). The crystals were recrystallized twice from ca. 9 ml methanol/acetone solvent (1:99) per gram of salt and dried at 80°C in vacuo for one day to give a purified crystalline product (6.00 g, 85% chem. yield, 96%de). IR(KBr): 2800-2200, 1720, 1610, 1265, 1105 $cm^{-1}$.
mp ca. 133°C (sintered), 145-148°C (dec.).

$[\alpha]_D^{21}$ +26° ($c$=1.0,$CHCl_3$)

Anal. calc'd for $C_{52}H_{57}NO_{12}\cdot H_2O$: C:68.93; H:6.56; N:1.55.
Found: C:69.12; H:6.37; N:1.63.

The purified crystals (5.50 g) were dissolved in 20 ml of ethanol and treated with 12.3 ml of N-NaOH and 40 ml $H_2O$. The resulting crystals were collected and recrystallized once from chloroform-ethanol (2:1) to yield the optically pure (96%ee) (R)-(+)-enantiomer (2.90 g, 79% chem. yield, calc'd as anhydrous). As the dried sample was very hygroscopic, it was allowed to equilibrate at atmospheric pressure and room temperature until constant weight was reached and then analyzed. mp 211-213°C.
IR (KBr): 1570 $cm^{-1}$.
$[\alpha]_D^{21}$ +33° (*c*=0.40, *CHCl*$_3$)
$^1$H-NMR [DMSO-$d^6$], δ; 7.50 (4H, d., J=6Hz, *o*-H of monosubstituted benzenes), 7.25 (4 H, s, disubstituted aromatic H), 7.0-7.4 (6H, m, p,m-H of monosubstituted benzenes), 5.1-5.3 (1H, m, OH or COOH), 3.0-5.0 (m, OH and/or COOH, overlapping with $H_2O$), 4.3-4.6 (1H, m, CH-OH), ca.2.80 (2H, bd. d, J=9Hz, equatorial H of N-$CH_2$ x2 in piperidine ring), 1.44 (6H, s, $CH_3$ x2), 1.0-2.4 (13H, m, remaining H).
Anal. calc'd for $C_{32}H_{39}NO_4 \cdot 1.2H_2O$: C:73.45; H:7.97; N:2.68.
Found: C:73.52; H:7.99; N:2.65.

Example 1B

(S)-(-)-4-[4-[4-(hydroxydiphenylmethyl)-1-piperidinyl]-1-hydroxybutyl]-α,α-dimethylbenzeneacetic acid

To the mother liquor from the crystallization of the (R)-(+)-enantiomer and (+)-di-para-toluoyltartaric acid was added 1N NaOH (15 ml) and 100 ml $H_2O$. The resulting crystals were collected and recrystallized once from chloroform-ethanol (2:1) to yield partially resolved (S)-(-)-4-[4-[4-(hydroxydiphenylmethyl)-1-piperidinyl]-1-hydroxybutyl]-α,α-dimethylbenzeneacetic acid (3.14 g, 79% chem. yield).

The crude (-)-enantiomeric crystals were combined with (2R,3R)-(-)-di-p-toluoyltartaric acid (2.42 g, 6.26 mmole) in acetone (45 ml) and remained in a refrigerator set to 4°C for 3 days. The resulting crystals were collected by filtration to yield the crude diastereomeric salt of the (S)-(-)-enantiomer with the resolving agent (4.81 g, 68% chem. yield). The salt was recrystallized once from a methanol/acetone solvent (1:99), mixed in a rough proportion of about 9 ml solvent per gram of salt, and dried at 80°C in vacuo for one day, yielding purified crystals (4.56 g, 65% chem. yield, 99%de).
mp. ca. 133°C (sintered), 146-149°C (dec.).
IR (KBr): 2800-2200, 1720, 1610, 1265, 1107 $cm^{-1}$.
$[\alpha]_D^{21}$ -26° (*c*=1.0,*CHCl*$_3$)
Anal. calc'd for $C_{52}H_{57}NO_{12} \cdot H_2O$: C:68.93; H:6.56; N:1.55.
Found: C:69.28; H:6.34; N:1.61.

The purified crystals (3.70 g) were dissolved in 15 ml of ethanol and treated with 8.3 ml of N-NaOH and 20 ml of $H_2O$. The resulting crystals were collected and recrystallized once from chloroform-ethanol (2:1) to yield the optically pure (99%ee) (S)-(-)-enantiomer (1.93 g, 60% chem. yield, calc'd as anhydrous). The sample was allowed to equilibrate prior to analysis. mp 211-213°C.
IR (KBr): 1570 $cm^{-1}$.
$[\alpha]_D^{21}$ -33° (*c*=0.41, *CHCl*$_3$)
$^1$NMR [DMSO-$d^6$], δ; 7.50 (4H, d, J=6Hz, *o*-H of monosubstituted benzenes), 7.25 (4H, s, disubstituted aromatic H), 7.0-7.4 (6H, *p,m*-H of monosubstituted benzenes), 5.1-5.3 (1H, m, OH or COOH), 3.0-5.0 (m, OH and/or COOH, overlapping with $H_2O$), 4.3-4.6 (1H, m, CH-OH), ca. 2.80 (2H, bd. d, J=9Hz, equatorial H of N-$CH_2$ x2 in the piperidine ring), 1.44 (6H, s, $CH_3$ x2), 1.0-2.4 (13H, m, remaining protons).
Anal. calc'd for $C_{32}H_{39}NO_4 \cdot 1.2H_2O$: C:73.45; H:7.97; N:2.68.
Found: C:73.38; H:7.99; N:2.64.

Example 2

(R)-(+)-4-[4-[4-(hydroxydiphenylmethyl)-1-piperidinyl]-1-hydroxybutyl]-α,α-dimethylbenzeneacetic acid

4-[4-[4-(hydroxydiphenylmethyl)-1-piperidinyl]-1-hydroxybutyl]-α,α-dimethylbenzeneacetic acid (500 mg, 1.0 mmole) and equimolar amounts of the resolving agent were dissolved into the organic solvent by heating to almost reflux temperature. This solution was cooled either at room temperature or in a refrigerator set to 4°C until crystals appeared and settled in the container. The crystals were collected over suction. Actual experimental results are reported in Table 3, while Table 4 gives the experimental conditions.

It is apparent after examination of Table 3 that (+)-di-para-toluoyltartaric acid was the only resolving agent tested which exhibits any measure of utility in resolving the (R)-(+)-enantiomer of the 4-α,α-dimethylbenzeneacetic acid derivative of terfenadine. It is also apparent that acetone is the most efficient organic solvent.

Table 3:

| Optical Resolution of 4-α,α-dimethylbenzene acetic acid Terfenadine Derivative with a Variety of Resolving Agents in Various Solvents | | | | | | | | |
|---|---|---|---|---|---|---|---|---|
| Example | Resolving Agent | Organic solvent | Diastereomer formed | Reaction yield (%)[a] | | Optical Purity (% de, ee) | | |
| | | | | | | %de[b] | | % ee[c] |
| | | | | 1 cryst | (X) recryst | 1 cryst | (x) recryst | (X) recryst |
| 1A | (+)-DPTTA·$H_2O$ | acetone | (+)-isomer/ (+)-DPTTA | 107 | (1x) 79 | 74 | (2x) 96 | (1x) 96 |
| 2A | (+)-DPTTA ·$H_2O$ | ethanoi | (+)-isomer/ (+)-DPTTA | 14 | -- | 46 | -- | -- |
| 2B | (+)-DPTTA ·$H_2O$ | 2-butanone | (+)-isomer/ (+)-DPTTA | 17 | -- | 86 | -- | -- |
| 2C | (-)-BNDHP | ethanol | none | -- | -- | -- | -- | -- |
| 2D | (-)-camphor-sulfonic acid | ethanol | none | -- | -- | -- | -- | -- |
| 2E | L-malic acid | ethanol | none | -- | -- | -- | -- | -- |
| 2F | (-)-M.A. | ethanol/ $H_2$0, 1:2 | racemic crystals[d] | -- | -- | 0 | -- | -- |
| 2G | (-)-M.A. | acetone | racemic crystals[d] | -- | -- | 0 | -- | -- |
| 2H | (-)-1-phenyl-ethylamine | MeOH/ EtOH, 1:1 | racemic crystals[d] | -- | -- | 0 | -- | -- |
| 2I | L-tartaric acid | ethanol | none | -- | -- | -- | -- | -- |

**KEY**

DPTTA = di-para-toluoyltartaric acid
M.A = mandelic acid
L-PCA = L-2-pyrrolidone-5-carboxylic acid
BNDHP = 1,1'-binaphthyl-2,2'-diyl hydrogen phosphate

[a]First column reflects chemical yield of crude diastereomeric complex after initial isolation. Second column reflects chemical yield of final purified enantiomer after separation and (x) recrystallizations.

[b]Optical purity is measured by chiral HPLC analysis. Diastereomeric complex measured both after initial isolation of diastereomeric salt in first column and after (x) recrystallizations in second column.

[c]Optical purity determined by chiral HPLC analysis. Enantiomeric excess determined after (x) recrystallizations of enantiomer after initial isolation from diastereomeric salt.

[d]Crystallization of both enantiomers

Table 4:

| Experimental Conditions for the Resolution of 4-α,α-dimethylbenzene acetic acid terfenadine deriviative | | | | | | |
|---|---|---|---|---|---|---|
| | Resolving Agent | | Organic solvent | | Reaction Conditions | |
| Example | type | amt (mg) | type | ml | Temp.[a] (°C) | Time (days) |
| 2A | (+)-DPTTA·$H_2O$ | 404 | ethanol | 4 | 4 | 10 |

**KEY**

DPTTA = di-para-toluoyltartaric acid
M.A. = mandelic acid
L-PCA = L-2-pyrrolidone-5-carboxylic acid
BNDHP = 1,1'-binaphthyl-2,2'-diyl hydrogen phosphate
[a]r.t. = room temperature (15°C to 30°C).

Table 4: (continued)

| Experimental Conditions for the Resolution of 4-α,α-dimethylbenzene acetic acid terfenadine deriviative | | | | | | |
|---|---|---|---|---|---|---|
| | Resolving Agent | | Organic solvent | | Reaction Conditions | |
| Example | type | amt (mg) | type | ml | Temp.[a] (°C) | Time (days) |
| 2B | (+)-DPTTA·$H_2O$ | 404 | 2-butanone | 2 | 4 | 10 |
| 2C | (-)-BNDHP | 348 | ethanol | 4 | r.t.[a] | 9 |
| 2D | (-)-camphorsulphonic acid | 232 | ethanol | 2 | r.t.[a] | 3 |
| 2E | L-malic acid | 134 | ethanol | 2 | r.t.[a] | 3 |
| 2F | (-)-M.A. | 152 | ethanol/$H_2O$, 1:2 | 12 | r.t.[a] | 4 |
| 2G | (-)-M.A. | 152 | acetone | 2 | 4 | 10 |
| 2H | (-)-1-phenyl-ethylamine | 121 | methanol/EtOH, 1:1 | 8 | 4 | 4 |
| 2I | L-tartaric acid | 150 | ethanol | 2 | r.t.[a] | 3 |

**KEY**

DPTTA = di-para-toluoyltartaric acid
M.A. = mandelic acid
L-PCA = L-2-pyrrolidone-5-carboxylic acid
BNDHP = 1,1'-binaphthyl-2,2'-diyl hydrogen phosphate
[a]r.t. = room temperature (15°C to 30°C).

In examining Table 3, it is realized that the use of the resolving agent (+)-DPTTA and the organic solvent acetone results in higher chemical yields and greater optical purity than any other resolving agent and organic solvent combination tested.

Resolution of ethyl 4-α,α-dimethylbenzeneacetate derivative

Reference Example 4A

(R)-(+)-ethyl 4-[4-[4-(hydroxydiphenylmethyl)-1-piperidinyl]-1-hydroxybutyl]-α,α-dimethylbenzeneacetate

Racemic ethyl 4-[4-[4-(hydroxydiphenylmethyl)-1-piperidinyl]-1-hydroxybutyl]-α,α-dimethylbenzeneacetate (10 g, 18.9 mmole) and (2S,3S)-(+)-di-p-toluoyltartaric acid monohydrate (7.64 g, 18.9 mmole) were dissolved in 80 ml of acetone by heating to ca. 55°C. The resulting solution was cooled to room temperature for one day and then in a refrigerator set to 4 °C for an additional day. The crystals were collected by filtration to yield the crude diastereomeric salt (98% chemical yield, 8.48 g). This material had an optical purity of 92% diastereomeric excess. The crude salt was recrystallized twice from ca. 6 ml acetone per gram of the salt and dried at 80°C in vacuo for one day resulting in purified diastereomeric salt (7.45 g, 86% chemical yield). The optical purity was determined to be 99% diastereomeric excess.

IR (KBr): 2800-2200, 1720, 1607, 1265, 1105 $cm^{-1}$. mp. ca. 113-120°C (hot stage).

$[\alpha]_D^{24}$ +20° (*c*= 1.0,*CHCl*$_3$)

Analysis calculated for $C_{54}H_{61}NO_{12}\cdot(0.5)H_2O$: C:70.11; H:6.76; N:1.51. Found: C:70.00; H:6.63; N:1.50.

The purified diastereomeric salt (6.95 g) was redissolved into 40 ml of ethanol and was subsequently treated with 15.5 ml of 1N NaOH and 25 ml of $H_2O$. The resulting crystals were collected and recrystallized once from ethanol/$H_2O$ (2:1) to yield the optically pure (99%ee) (R)-(+)-enantiomer. (3.93 g, 84% chemical yield). mp. 141-142°C.

IR (KBr): 1727, 1707 $cm^{-1}$

$^1$H-NMR ($CDCl_3$), δ; 7.1-7.6 (14H, m, aromatic H), 4.5-4.7 (1H, m, <u>CH</u>-OH), 4.09 (2H, quart., J=7.0Hz, <u>$CH_2$</u>$CH_3$), ca. 3.06 (2H, bd. trip., J=13Hz, axial H of N-$CH_2$ x2 in the piperidine ring), 1.4-2.6 (14H, m, remaining H), 2.23 (1H, s, OH), 1.54 (6H, s, $CH_3$ x2), 1.15 (3H, trip., J=7.0Hz, $CH_2$<u>$CH_3$</u>)

$[\alpha]_D^{24}$ +49°;(*c*= 1.0,*CHCl*$_3$)

Analysis calculated for $C_{34}H_{43}NO_4$: C:77.09; H:8.18; N:2.64. Found C:76.88; H:8.29; N:2.55.

Table 5 graphically illustrates the experimental results along with certain reaction parameters, permitting a comparison with other resolving agents and organic solvents.

Reference Example 4B

(S)-(-)-ethyl 4-[4-[4-(hydroxydiphenylmethyl)-1-piperidinyl]-1-hydroxybutyl]-α,α-dimethylbenzeneacetate

To the mother liquor remaining from the crystallization of the (R)-(+)-enantiomer and (+)-di-p-toluoyltartaric acid was added 20 ml of 1N NaOH and 70 ml of $H_2O$. The resulting crystals were collected and recrystallized once from ethanol/$H_2O$ (2:1) and yielded partially resolved (S)-(-)-ethyl 4-[4-[4-(hydroxydiphenylmethyl)-1-piperidinyl]-1-hydroxybutyl]-α,α-dimethylbenzeneacetate (4.96 g, 99% chemical yield).

The crude crystalline material and (2R,3R)-(-)-di-paratoluoyltartaric acid ((-)-DPTTA)(3.62 g, 9.37 mmole) were mixed into a solution with 50 ml acetone and remained at room temperature (15°C to 30°C) for one day and then in a refrigerator set to 4°C for an additional day. The resulting crystals were collected by filtration to yield a crude diastereomeric salt of the (S)-(-)-enantiomer and (-)-DPTTA (7.64 g, 88% chemical yield). The salt was recrystallized once from ca. 6 ml acetone per gram of salt and dried at 80°C in vacuo for one day to give purified diastereomeric salt. (7.25 g, 84% yield, 99%de). mp. ca. 113-120°C (hot stage).
IR (KBr): 2800-2200, 1720,1607,1265,1105 $cm^{-1}$.

$[\alpha]_D^{24}$ -21° ($c$=1.0, $CHCl_3$)

Analysis calculated for $C_{54}H_{61}NO_{12}\cdot(0.5)H_2O$: C:70.11; H:6.76; N:1.51. Found: C:70.19; H:6.69; N:1.52.

To the solution of 6.75 g of the purified diasteromeric salt in 40 ml of ethanol was added 15.0 ml of 1N NaOH and then 25 ml of $H_2O$. The resulting crystals were collected and recrystallized once from ethanol/$H_2O$ (2:1) and yielded optically pure (99%ee) (S)-(-)-ethyl 4-[4-[4-(hydroxydiphenylmethyl)-1-piperidinyl]-1-hydroxybutyl]-α,α-dimethylbenzeneacetate. (3.82 g, 82% chemical yield)
IR (KBr): 1727,1707 $cm^{-1}$. mp. 141-142°C.

$[\alpha]_D^{24}$ -48°; ($c$=1.0, $CHCl_3$)

$^1$H-NMR ($CDCl_3$), δ; 7.1-7.6 (14H, m, aromatic H), 4.5-4.7 (1H, m, CH-OH), 4.09 (2H, quart., J=7.0Hz, $CH_2CH_3$), ca. 3.06 (2H, bd. trip., J=13Hz, axial H of N-$CH_2$ x2 in the piperidine ring), 1.4-2.6 (14H, m, remaining H), 2.23 (1H, s, OH), 1.54 (6H, s, $CH_3$ x2), 1.15 (3H, trip., J=7.0Hz, $CH_2CH_3$)
Analysis calculated for $C_{34}H_{43}NO_4$: C:77.09; H:8.18; N:2.64.
Found: C:76.86; H:8.47; N:2.61.

Reference Example 5A

(R)-(+)-ethyl 4-[4-[4-(hydroxydiphenylmethyl)-1-piperidinyl]-1-hydroxybutyl]-α,α-dimethylbenzeneacetate

Racemic ethyl 4-[4-[4-(hydroxydiphenylmethyl)-1-piperidinyl]-1-hydroxybutyl]-α,α-dimethylbenzeneacetate (20 g, 37.8 mmole) and (R)-(-)-mandelic acid (5.75 g, 37.8 mmole) were dissolved in 110 ml of methanol by heating to ca. 60 °C. The resulting solution remained at room temperature (15°C to 30°C) for one day and then in a refrigerator set to 4°C for an additional day. The resulting crystals were collected by filtration to yield crystalline diastereomeric salt (12.3 g, 95% yield, 82%de) comprising the (R)-(+)-enantiomer and (R)-(-)-mandelic acid. The crystals were recrystallized twice from ca. 6 ml methanol per gram of diastereomeric salt and dried at 50°C in vacuo for one day to give purified diastereomeric salt (8.90 g, 69% yield, 99%de). mp. ca. 73°C (sintered), ca. 78-83°C (hot stage).
IR (KBr): 2800-2100, 1727, 1607, 1360 $cm^{-1}$.

$[\alpha]_D^{22}$ -4.9° ($c$= 2.0, $CHCl_3$)

Analysis calculated for $C_{42}H_{51}NO_7\cdot(0.25)H_2O$: c:73.50; H:7.56; N:2.04. Found: C:73.38; H:7.62; N:2.06.

The purified diastereomeric salt (8.40 g) was dissolved into 50 ml of ethanol and was treated with 1N NaOH (12.5 ml) and $H_2O$ (40 ml). The crystals were collected and recrystallized once from ethanol/$H_2O$ (2:1) to give optically pure (99%ee) (R)-(+)-ethyl 4-[4-[4-(hydroxydiphenylmethyl)-1-piperidinyl]-1-hydroxybutyl]-α,α-dimethylbenzeneacetate (6.08 g, 64% yield). mp. 140-141°C.

$[\alpha]_D^{22}$ +48° ($c$= 1.0,$CHCl_3$)

IR (KBr): 1727, 1707 $cm^{-1}$. Analysis calculated for $C_{34}H_{43}NO_4$: C:77.09; H:8.18; N:2.64. Found: C:76.93; H:8.31; N: 2.56.

Table 5 graphically illustrates the experimental results along with certain reaction parameters, permitting a comparison with other resolving agents and organic solvents.

Reference Example 5B

(S)-(-)-ethyl 4-[4-[4-(hydroxydiphenylmethyl)-1-piperidinyl]-1-hydroxybutyl]-α,α-dimethylbenzeneacetate

The filtrate from the crystallization of the crude diastereomeric salt between (R)-(+)-enantiomer with (R)-(-)-mandelic acid was treated with 1N NaOH (20 ml) and $H_2O$ (50 ml). The resulting crystals were collected and recrys-

tallized once from ethanol/$H_2O$ (2:1) to give the partially resolved (S)-(-)-enantiomer. (10.4 g, 100.4% yield).

A solution was formed comprising the crystalline (S)-(-)-enantiomer (19.6 mmole) and (S)-(+)-mandelic acid (2.99 g, 19.7 mmol) in methanol (75 ml) and remained at room temperature (15°C to 30°C) for one day and then in a refrigerator set to 4°C for another day. The crystalline material was then collected by filtration to give crystalline diastereomeric salt comprising the (S)-(-)-enantiomer and (S)-(+)-mandelic acid. (10.2 g, 79% yield). The crystals were recrystallized once from ca. 6 ml methanol per gram of the salt and dried at 50°C in vacuo for one day to give the purified diastereomeric salt (9.07 g, 70% yield). mp. ca. 72°C (sintered), ca. 77-83°C (hot stage).

$[\alpha]_D^{22}$ +4.8° (*c*= 2.0, *$CHCl_3$*)

IR (KBr): 2800-2100, 1727, 1607, 1360 $cm^{-1}$

Analysis calculated for $C_{42}H_{51}NO_7$: C:73.98; H:7.54; N:2.05.

Found: C:73.84; H:7.58; N:2.09.

The purified salt (8.50 g) was dissolved into 50 ml of ethanol and subsequently treated with 1N NaOH (12.7 ml) and then $H_2O$ (40 ml). The crystals were collected and recrystallized from ethanol/$H_2O$ (2:1) to yield optically pure (98%ee) (S)-(-)-ethyl 4-[4-[4-(hydroxydiphenylmethyl)-1-piperidinyl]-1-hydroxybutyl]-α,α-dimethylbenzeneacetate (6.11 g, 64% yield). mp. 141-142°C.

IR (KBr): 1727, 1707 $cm^{-1}$.

$[\alpha]_D^{22}$ -48° (*c*= 1.0,*$CHCl_3$*)

Analysis calculated for $C_{34}H_{43}NO_4$: C:77.09; H:8.18; N:2.64.

Found: C:77.33; H:8.41; N:2.64.

Reference Example 6

(R)-(+)-ethyl 4-[4-[4-(hydroxydiphenylmethyl)-1-piperidinyl]-1-hydroxybutyl]-α,α-dimethylbenzeneacetate

Ethyl 4-[4-[4-(hydroxydiphenylmethyl)-1-piperidinyl]-1-hydroxybutyl]-α,α-dimethylbenzeneacetate (500 mg, 0.94 mmole) and equimolar amounts of the resolving agent were added together into the organic solvent and dissolved by heating to almost refluxing temperature. The solution was cooled either to room temperature or at 4°C in a refrigerator for a period of time. The resulting crystals were dried over a suction. The results are presented in tabular form in Table 5, and the individual experimental conditions in Table 6.

Comparative Example 2A

(S)-(-)-ethyl 4-[4-[4-(hydroxydiphenylmethyl)-1-piperidinyl]-1-hydroxybutyl]-α,α-dimethylbenzeneacetate

Racemic ethyl 4-[4-[4-(hydroxydiphenylmethyl)-1-piperidinyl]-1-hydroxybutyl]-α,α-dimethylbenzeneacetate (45.0 g, 85.0 mmol) and (R)-(-)-1,1'-binaphthyl-2-2'-diyl hydrogen phosphate ((R)-(-)-BNDHP) were dissolved into 300 ml of 2-butanone and heated to form a solution. The solution remained at room temperature (15°C to 30°C) for 3 days and the crystals were collected by filtration. The crystals were then dissolved in about 100 ml of hot methanol and then concentrated. The oily residue was then dissolved in ca. 100 ml of 2-butanone and concentrated. Finally, the remaining oily residue was dissolved in 100 ml of hot 2-butanone and then cooled to room temperature (15°C to 30°C) for 20 hours. The hot methanol/2-butanone procedure was repeated an additional seven times to yield the purified diastereomeric salt of the (S)-(-)-enantiomer and (R)-(-)-1,1'-binaphthyl-2,2'-diyl hydrogen phosphate (21.6 g).

The salt was suspended in 60 ml of ethanol and treated with 1N NaOH (30 ml) and remained at room temperature overnight (20 hours). The resulting crystals were collected by filtration and recrystallized from ethanol/water (2:1) to yield the title compound. (12.4 g, 55% yield). mp. 139-140°C.

$[\alpha]_D^{23}$ -48° (*c*= 1.05, *$CHCl_3$*)

Analysis calculated for $C_{34}H_{43}NO_4$: C:77.09; H:8.18; N:2.64.

Found: C:77.15; H:8.20; N:2.63.

Table 5 graphically illustrates the experimental results along with certain reaction parameters, permitting a comparison with other resolving agents and organic solvents.

Comparative Example 2B

(R)-(+)-ethyl 4-[4-[4-(hydroxydiphenylmethyl)-1-piperidinyl]-1-hydroxybutyl]-α,α-dimethylbenzeneacetate

The filtrate from the crystallization of the (S)-(-)-enantiomer and (R)-(-)-BNDHP and the washings were combined and concentrated. The oily residue was dissolved in a mixture of ethanol (140 ml) and 1N NaOH (70 ml) and remained at room temperature (15°C to 30°C). The crude crystalline product was recrystallized from ethanol/water (2:

1) (24.3 g).

$[\alpha]_D^{23}$ +25° ($c$= 1.07, $CHCl_3$)

The crude crystalline product was combined with (S)-(+)-1,1'-binaphthyl-2,2'-diyl hydrogen phosphate ((S)-(+)-BNDHP) into 200 ml of 2-butanone and heated to form a solution. The solution remained at room temperature (15°C to 30°C) for four days after which the resulting crystals were redissolved in hot methanol and concentrated. The remaining oily residue was concentrated and dissolved in ca. 100 ml 2-butanone and concentrated. Finally, the oily residue was dissolved in 100 ml of hot 2-butanone and then cooled to room temperature (15°C to 30°C) for 20 hours. The methanol/2-butanone recrystallizations were repeated seven additional times yielding the diastereomeric salt of the (R)-(+)-enantiomer and (S)-(+)-BNDHP (18.7 g).

The diastereomeric salt was suspended in 60 ml of ethanol and treated with 1N NaOH (30 ml) and remained at room temperature (15°C to 30°C) overnight (20 hours). The resulting crystals were recrystallized from ethanol/water (2:1) yielding the title compound (10.2 g, 45% yield). mp. 139-140°C.

$[\alpha]_D^{23}$ +48° ($c$= 1.06, $CHCl_3$)

Analysis calculated for $C_{34}H_{43}NO_4$: C:77.09; H:8.18; N:2.64.
Found C:77.00; H:8.20; N:2.64.

Table 5 :

| Optical Resolution of ethyl 4-α,α-dimethylbenzene acetate terfenadine derivative | | | | | | | |
|---|---|---|---|---|---|---|---|
| Reference Example | Resolving Agent | Organic solvent | Diastereomer formed | Reaction yield (%)[a] | | Optical Purity (% de, ee)[b] | |
| | | | | 1 cryst | (X) recryst | 1 cryst | (X) recryst |
| 4A | (-)-DPTTA·$H_2O$ | acetone | (+)-isomer/ (+)-DPTTA | 98 | (1x) 84 | 92 | (1x) 99 |
| 5A | (-)-M.A. | methanol | (+)-isomer/(-). M.A. | 95 | (1x) 64 | 82 | (1x) 99 |
| 6A | abietic acid | ethanol | none | -- | -- | -- | -- |
| 6B | (-)-BNDHP | methanol | none | -- | -- | -- | -- |
| 6C | (-)-BNDHP | EtOH/$H_2O$ 2:1 | none | -- | -- | -- | -- |
| 6D | (+)-camphoric acid | ethanol | none | -- | -- | -- | -- |
| 6E | (-)-camphorsulphonic acid | ethanol | none | -- | -- | -- | -- |
| 6F | (+)-DPTTA·$H_2O$ | ethanol | (+)-isomer/ (+)-DPTTA | 71 | -- | 54 | -- |
| 6G | L-malic acid | ethanol | none | -- | -- | -- | -- |
| 6H | (-)-M.A. | ethanol | (+)-isomer/ (-)-M.A. | 91 | -- | 78 | -- |
| 6I | (-)-M.A. | acetone | none | -- | -- | -- | -- |
| 6J | (-)-M.A. | $CH_3CO_2Et$ | none | -- | -- | -- | -- |
| 6K | (-)-M.A. | 2-butanone | none | -- | -- | -- | -- |

**KEY**

DPTTA = di-para-toluoyltartaric acid
M.A. = mandelic acid
L-PCA = L-2-pyrrolidone-5-carboxylic acid
BNDHP = 1,1'-binaphthyl-2,2'-diyl hydrogen phosphate

[a]First column reflects chemical yield after initial isolation of diastereomeric salt. Second column reflects chemical yield after (x) recrystallizations of separated, purified enantiomer.

[b]First column reflects optical purity in diastereomeric excess after initial isolation of the diastereomeric complex. Second column reflects optical purity in enantiomeric excess after (x) recrystallizations of the isolated enantiomer.

Table 5 : (continued)

| Optical Resolution of ethyl 4-$\alpha,\alpha$-dimethylbenzene acetate terfenadine derivative | | | | | | | |
|---|---|---|---|---|---|---|---|
| Reference Example | Resolving Agent | Organic solvent | Diastereomer formed | Reaction yield (%)[a] | | Optical Purity (% de, ee)[b] | |
| | | | | 1 cryst | (X) recryst | 1 cryst | (X) recryst |
| 6L | (-)-M.A. | $CH_3CN$ | none | -- | -- | -- | -- |
| 6M | (-)-M.A. | dioxane | none | -- | -- | -- | -- |
| 6N | L-PCA | ethanol | none | -- | -- | -- | -- |
| 6O | L-tartaric acid | ethanol | none | -- | -- | -- | -- |
| Comp. 2A | (-)-BNDHP | MeOH/ 2-butanone | (-)-isomer/(-)-BNDHP | 131 | (1x) 55 | 30 | (1x) 98 |

**KEY**

DPTTA = di-para-toluoyltartaric acid
M.A. = mandelic acid
L-PCA = L-2-pyrrolidone-5-carboxylic acid
BNDHP = 1,1'-binaphthyl-2,2'-diyl hydrogen phosphate

[a]First column reflects chemical yield after initial isolation of diastereomeric salt. Second column reflects chemical yield after (x) recrystallizations of separated, purified enantiomer.

[b]First column reflects optical purity in diastereomeric excess after initial isolation of the diastereomeric complex. Second column reflects optical purity in enantiomeric excess after (x) recrystallizations of the isolated enantiomer.

Table 6:

| Experimental Conditions for Resolution of Ethyl $\alpha,\alpha$-Dimethylbenzeneacetate Terfenadine Derivative | | | | | | |
|---|---|---|---|---|---|---|
| Reference Example | Resolving Agent | | Organic solvent solvent | | Reaction Conditions | |
| | type | amt (mg) | type | ml | Temp.[a] (°C) | Time (days) |
| 6A | abietic acid | 284 | ethanol | 2 | r.t. | 3 |
| 6B | (-)-BNDHP | 327 | methanol | 3 | r.t. | 2 |
| 6C | (-)-BNDHP | 327 | EtOH/ $H_2O$, 2:1 | 6 | r.t. | 2 |
| 6D | (+)-camphoric acid | 190 | ethanol | 2 | r.t. | 3 |
| 6E | (-)-camphorsulfonic acid | 218 | ethanol | 2 | r.t. | 3 |
| 6F | (+)-DPTTA·$H_2O$ | 388 | ethanol | 4 | 4 | 4 |
| 6G | L-malic acid | 126 | ethanol | 2 | r.t. | 3 |
| 6H | (-)-M.A. | 150 | ethanol | 5 | r.t. | 2 |
| 6I | (-)-M.A. | 150 | acetone | 3 | r.t. | 10 |
| 6J | (-)-M.A. | 150 | ethyl acetate | 2 | r.t. | 8 |
| 6K | (-)-M.A. | 150 | 2-butanone | 2 | r.t. | 8 |
| 6L | (-)-M.A. | 150 | methyl cyanide | 2 | r.t. | 8 |
| 6M | (-)-M.A. | 150 | dioxane | 2 | r.t. | 8 |

**KEY**

M.A. = mandelic acid
L-PCA = L-2-pyrrolidone-5-carboxylic acid
BNDHP = 1,1'-binaphthyl-2,2'-diyl hydrogen phosphate
[a]r.t. = room temperature (15°C to 30 °C).

Table 6: (continued)

| Experimental Conditions for Resolution of Ethyl $\alpha,\alpha$-Dimethylbenzeneacetate Terfenadine Derivative | | | | | | |
|---|---|---|---|---|---|---|
| Reference Example | Resolving Agent | | Organic solvent solvent | | Reaction Conditions | |
| | type | amt (mg) | type | ml | Temp.[a] (°C) | Time (days) |
| 6N | L-PCA | 121 | ethanol | 2 | r.t. | 8 |
| 6O | L-tartaric acid | 140 | ethanol | 2 | r.t. | 8 |

**KEY**
M.A. = mandelic acid
L-PCA = L-2-pyrrolidone-5-carboxylic acid
BNDHP = 1,1'-binaphthyl-2,2'-diyl hydrogen phosphate
[a]r.t. = room temperature (15°C to 30 °C).

In examining Table 5, it is evident that the use of the resolving agents (+)-DPTTA and (-)-mandelic acid resulting in resolution of greater chemical yield and higher optical purity, in fewer recrystallizations than the other resolving agents tested.

## Claims

**1.** A process for preparing a compound of a formula:

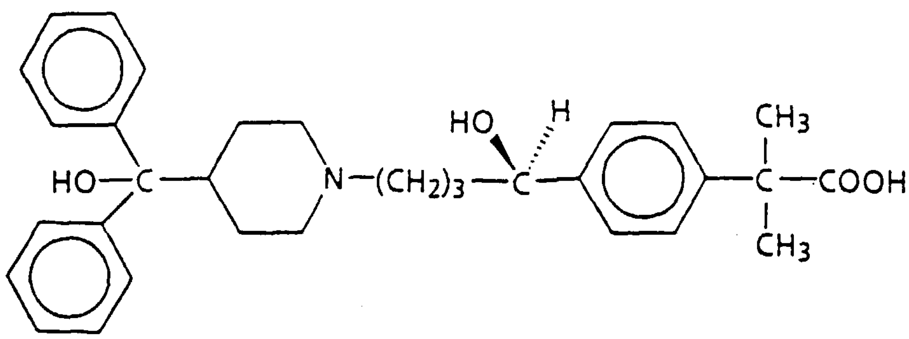

comprising:

a) dissolving into a solution an amount of a racemic compound of a formula:

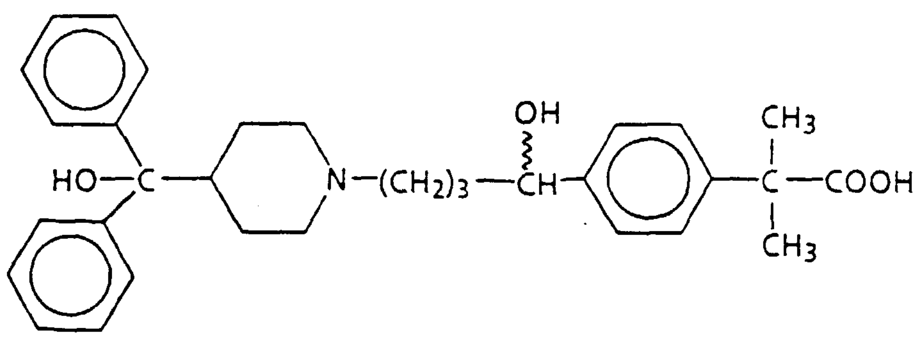

with an equimolar amount of an optically active resolving agent, (+)-di-para-toluoyltartaric acid, into a suitable organic solvent;
b) heating the solution to an elevated temperature suitable for formation of a solubilized diastereomeric salt between the optically active resolving agent and the compound;
c) cooling the solution for a period of time sufficient to precipitate the diastereomeric salt;

d) collecting the diastereomeric salt; and
e) hydrolysing the diastereomeric salt to isolate the compound.

**2.** A process for preparing a compound of a formula:

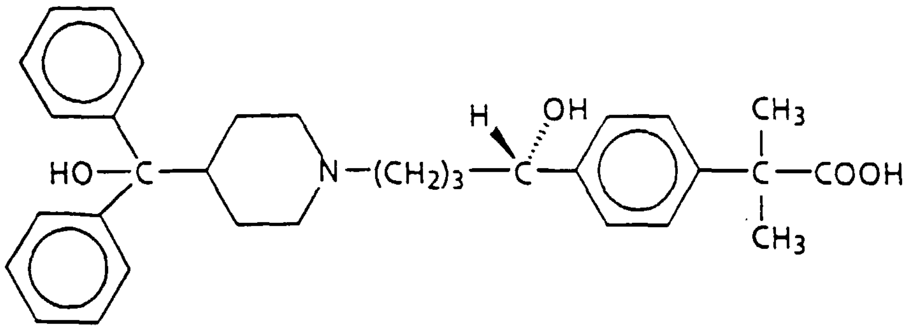

comprising:

a) dissolving into a solution an amount of a racemic compound of a formula:

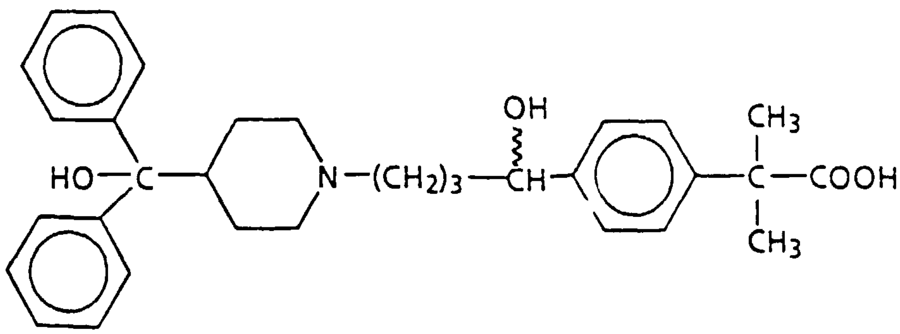

with an equimolar amount of optically active resolving agent, (-)-di-para-toluoyltartaric acid, into a suitable organic solvent;
b) heating the solution to an elevated temperature suitable for the formation of a solubilized diastereomeric salt between the optically active resolving agent and the compound;
c) cooling the solution for a period of time sufficient to precipitate the interactive complex as a diastereomeric salt;
d) collecting the diastereomeric salt; and
e) hydrolysing the diastereomeric salt to isolate the compound.

**3.** A process for preparing a compound of a formula:

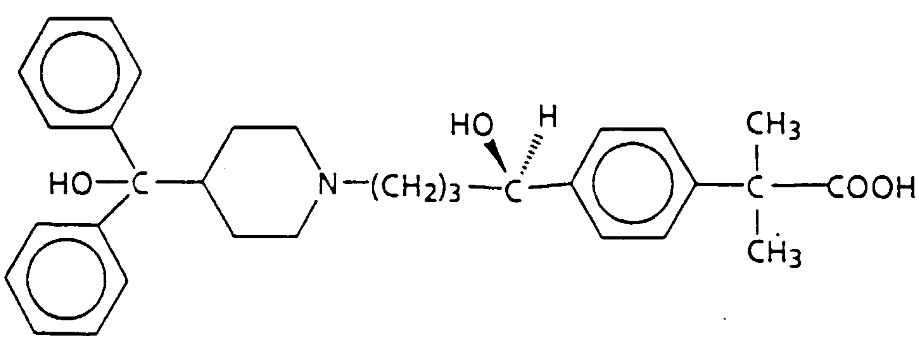

comprising:

a) dissolving into a solution an amount of a racemic compound of a formula:

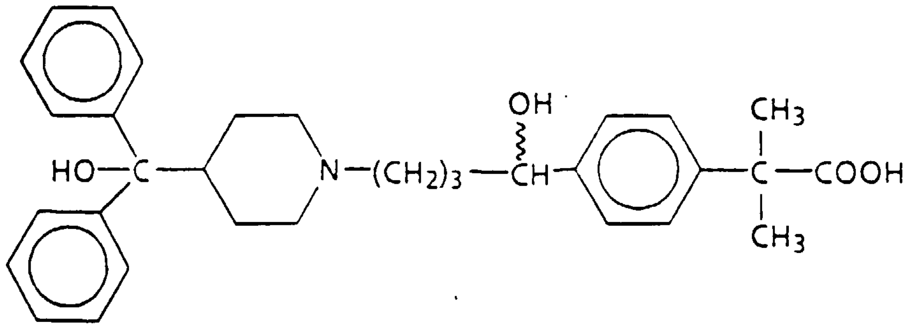

with an equimolar amount of an optically active resolving agent, (-)-di-para-toluoyltartaric acid, into a suitable organic solvent;
b) heating the solution to an elevated temperature suitable for formation of a solubilized first diastereomeric salt between the optically active resolving agent and the compound;
c) cooling the solution for a period of time sufficient to precipitate the first diastereomeric salt;
d) removing the first diastereomeric salt and preserving the solution as a filtrate;
e) hydrolysing and separating the compound from the filtrate;
f) dissolving into solution the compound with an optically active resolving agent', (+)-di-para-toluoyltartaric acid in an amount equimolar to an amount of the compound in such manner as to form a solubilized second diastereomeric salt between the same;
g) precipitating the second diastereomeric salt;
h) collecting the second diastereomeric salt; and
i) hydrolysing the second diastereomeric salt to isolate the compound.

**4.** A process for preparing a compound of a formula:

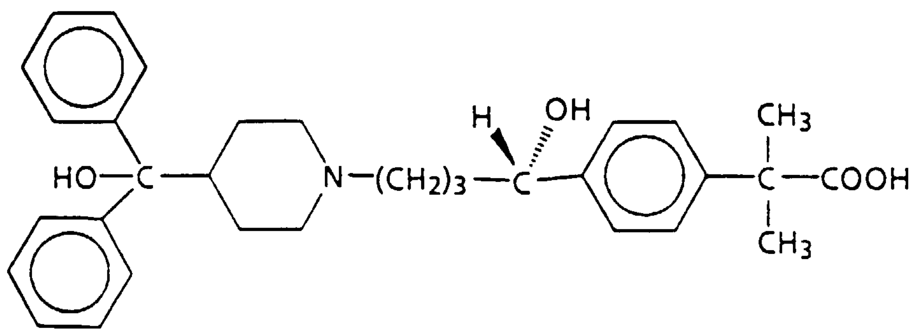

comprising:

a) dissolving into a solution an amount of a racemic compound of a formula:

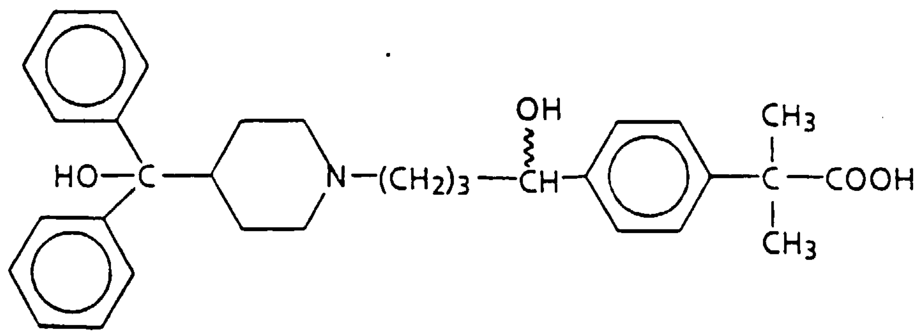

with an equimolar amount of an optically active resolving agent, (+)-di-para-toluoyltartaric acid, into a suitable organic solvent;

b) heating the solution to an elevated temperature suitable for formation of a solubilized first diastereomeric salt between the optically active resolving agent and the compound;

c) cooling the solution for a period of time sufficient to precipitate the first diastereomeric salt;

d) removing the first diastereomeric salt and preserving the solution as a filtrate;

e) hydrolysing and separating the'compound from the filtrate;

f) dissolving into solution the compound with an optically active resolving agent', (-)-di-para-toluoyltartaric acid, in an amount equimolar to an amount of the compound in such manner as to form a solubilized second diastereomeric salt between the same;

g) precipitating the second diastereomeric salt;

h) collecting the second diastereomeric salt; and

i) hydrolysing the second diastereomeric salt to isolate the compound.

**5.** A compound consisting essentially of a diastereomeric salt between (R)-(+)-4-[4-[4-(hydroxydiphenylmethyl)-1-piperidinyl]-1-hydroxybutyl]-α,α-dimethylbenzeneacetic acid and (2S,3S)-(+)-di-para-toluoyltartaric acid; or

a compound consisting essentially of a diastereomeric salt between (S)-(-)-4-[4-[4-(hydroxydiphenylmethyl)-1-piperidinyl]-1-hydroxybutyl]-α,α-dimethylbenzeneacetic acid and (2R,3R)-(-)-di-paratoluoyltartaric acid.

## Patentansprüche

**1.** Verfahren zur Herstellung einer Verbindung der Formel:

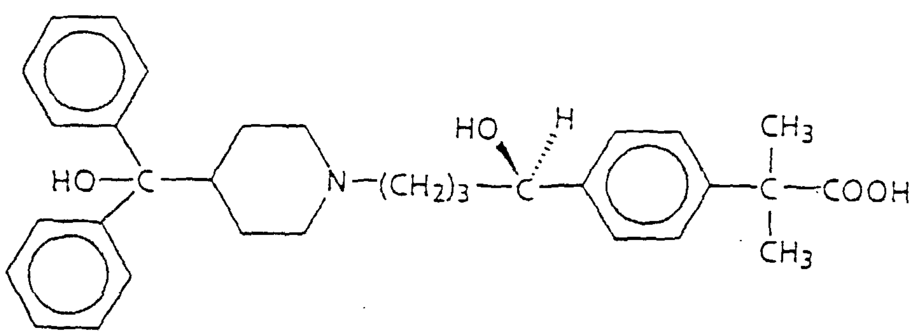

umfassend:

a) In Lösung bringen einer Menge einer racemischen Verbindung der Formel:

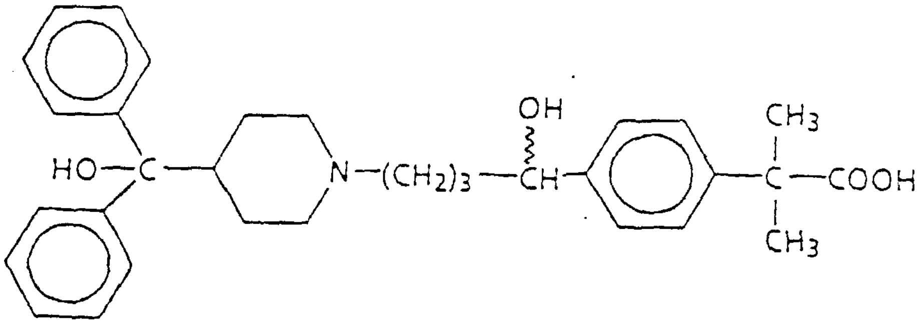

mit einer äquimolaren Menge eines optisch aktiven Trennmittels, (+)-Di-para-toluoyl-weinsäure, in einem geeigneten organischen Lösungsmittel;

b) Erwärmen der Lösung auf eine erhöhte Temperatur, die geeignet ist, ein solubilisiertes diastereomeres Salz aus dem optisch aktiven Trennmittel und der Verbindung zu erzeugen;

c) Abkühlen der Lösung für einen Zeitabschnitt, der ausreicht, um das diastereomere Salz auszufällen;

d) Auffangen des diastereomeren Salzes; und

e) Hydrolisieren des diastereomeren Salzes zum Isolieren der Verbindung.

**2.** Verfahren zur Herstellung einer Verbindung der Formel:

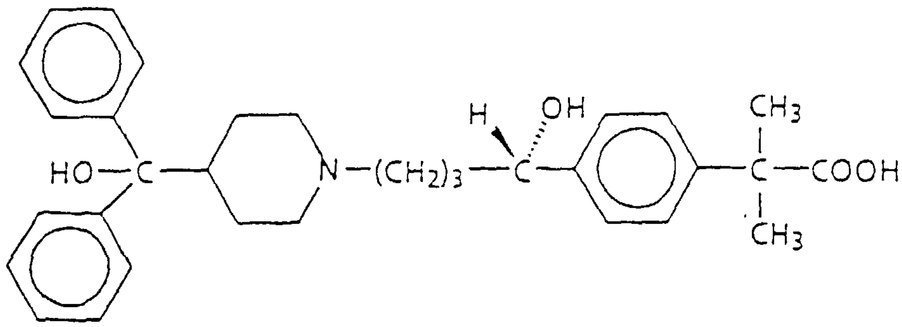

umfassend:

a) In Lösung bringen einer Menge einer racemischen Verbindung der Formel:

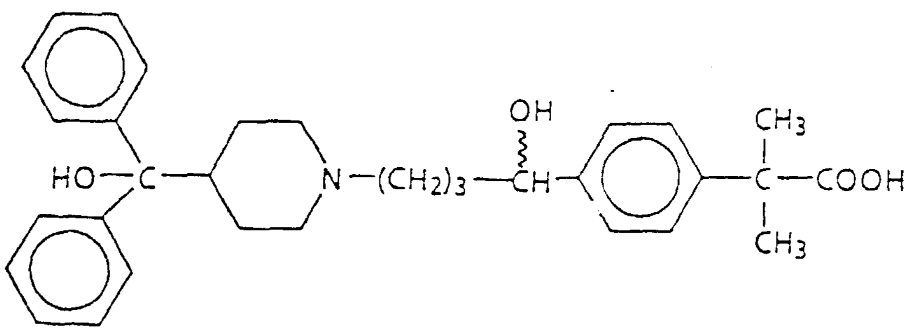

mit einer äquimolaren Menge eines optisch aktiven Trennmittels, (-)-Di-para-toluoyl-weinsäure, in einem geeigneten organischen Lösungsmittel;

b) Erwärmen der Lösung auf eine erhöhte Temperatur, die geeignet ist, ein solubilisiertes diastereomeres Salz aus dem optisch aktiven Trennmittel und der Verbindung zu erzeugen;

c) Abkühlen der Lösung für einen Zeitabschnitt, der ausreicht, um den interaktiven Komplex als diastereomeres

Salz auszufällen;
d) Auffangen des diastereomeren Salzes; und
e) Hydrolisieren des diastereomeren Salzes zum Isolieren der Verbindung.

**3.** Verfahren zur Herstellung einer Verbindung der Formel:

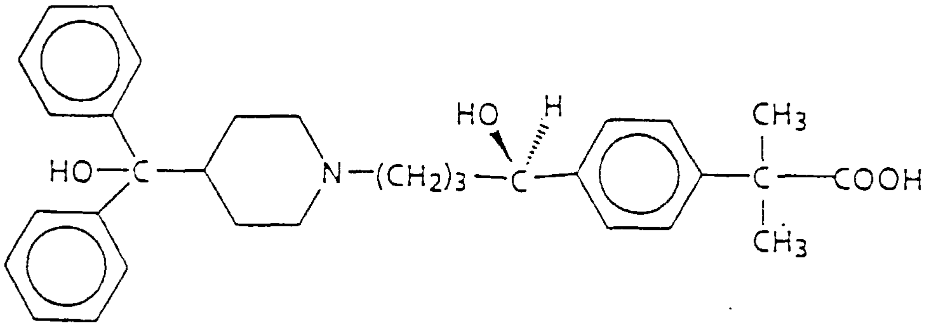

umfassend:

a) In Lösung bringen einer Menge einer racemischen Verbindung der Formel:

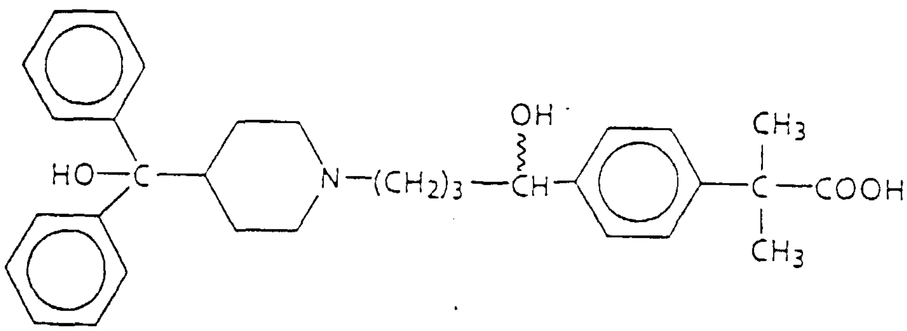

mit einer äquimolaren Menge eines optisch aktiven Trennmittels, (-)-Di-para-toluoyl-weinsäure, in einem geeigneten organischen Lösungsmittel;
b) Erwärmen der Lösung auf eine erhöhte Temperatur, die geeignet ist, ein solubilisiertes erstes diastereomeres Salz aus dem optisch aktiven Trennmittel und der Verbindung zu erzeugen;
c) Abkühlen der Lösung für einen Zeitabschnitt, der ausreicht, um das erste diastereomere Salz auszufällen;
d) Entfernen des ersten diastereomeren Salzes und Erhalten der Lösung als ein Filtrat;
e) Hydrolysieren und Abtrennen der Verbindung vom Filtrat;
f) Auflösen der Verbindung mit einem optisch aktiven Trennmittel , (+)-Di-para-toluoyl-weinsäure in einer Menge, die äquimolar zu einer Menge der Verbindung ist, und zwar dergestalt, daß ein zweites solubilisiertes diastereomeres Salz aus denselben erzeugt werden kann;
g) Ausfällen des zweiten diastereomeren Salzes;
h) Sammeln des zweiten diastereomeren Salzes; und
i) Hydrolisieren des zweiten diastereomeren Salzes zum Isolieren der Verbindung.

**4.** Verfahren zur Herstellung einer Verbindung der Formel:

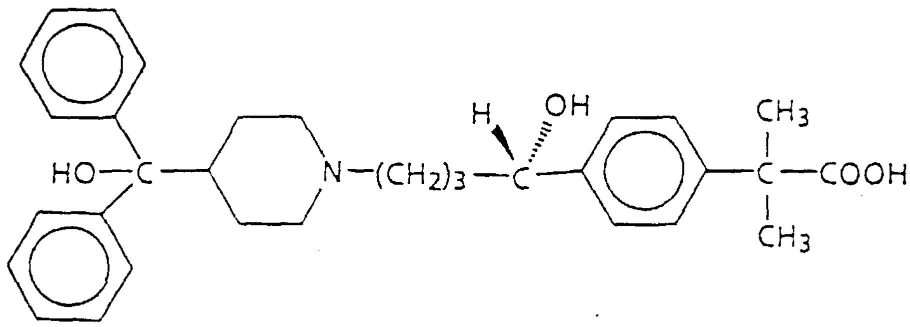

umfassend:

a) In Lösung bringen einer Menge einer racemischen Verbindung der Formel:

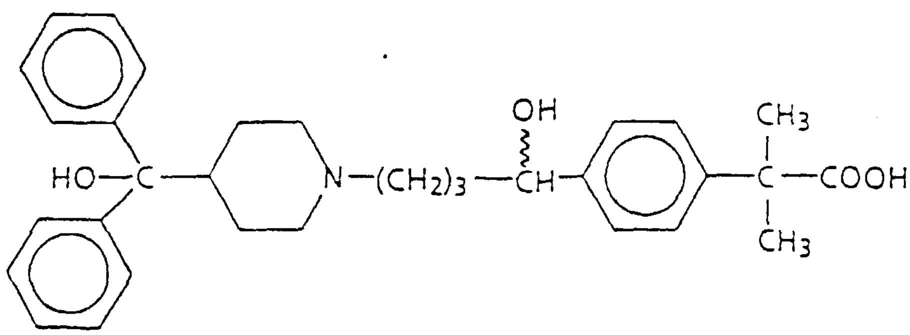

mit einer äquimolaren Menge eines optisch aktiven Trennmittels, (+)-Di-para-toluoyl-weinsäure, in einem geeigneten organischen Lösungsmittel;
b) Erwärmen der Lösung auf eine erhöhte Temperatur, die geeignet ist, ein solubilisiertes erstes diastereomeres Salz aus dem optisch aktiven Trennmittel und der Verbindung zu erzeugen;
c) Abkühlen der Lösung für einen Zeitabschnitt, der ausreicht, um das erste diastereomere Salz auszufällen;
d) Entfernen des ersten diastereomeren Salzes und Erhalten der Lösung als ein Filtrat;
e) Hydrolysieren und Abtrennen der Verbindung vom Filtrat;
f) Auflösen der Verbindung mit einem optisch aktiven Trennmittel, (-)-Di-para-toluoyl-weinsäure in einer Menge, die äquimolar zu einer Menge der Verbindung ist, und zwar dergestalt, daß ein zweites solubilisiertes diastereomeres Salz aus denselben erzeugt werden kann;
g) Ausfällen des zweiten diastereomeren Salzes;
h) Sammeln des zweiten diastereomeren Salzes;
i) Hydrolisieren des zweiten diastereomeren Salzes zum Isolieren der Verbindung.

**5.** Verbindung im wesentlichen bestehend aus einem diastereomeren Salz aus (R)-(+)-4-[4-[4-(Hydroxydiphenylmethyl)-1-piperidinyl]-1-hydroxybutyl]-α,α-dimethylbenzolessigsäure und (2S, 3S)-(+)-Di-para-toluoyl-weinsäure; oder eine Verbindung, im wesentlichen bestehend aus einem diastereomeren Salz aus (S)-(-)-4-[4-[4-(Hydroxydiphenylmethyl)-1-piperidinyl]-1-hydroxybutyl]-α,α-dimethylbenzolessigsäure und (2R, 3R)-(-)-Di-para-toluoylweinsäure.

## Revendications

**1.** Procédé pour préparer un composé de formule :

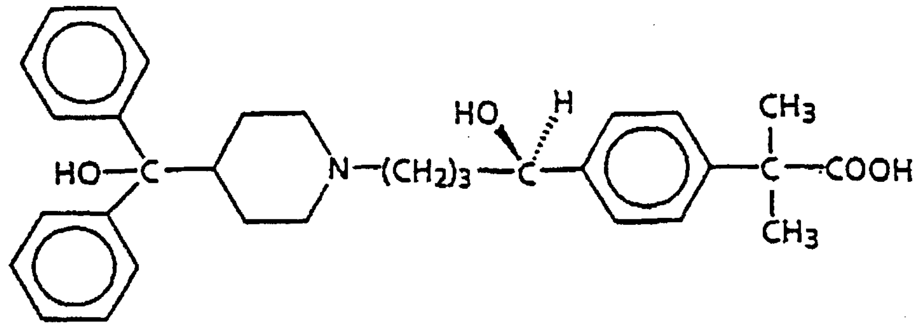

comprenant :

a) la dissolution dans une solution d'une quantité d'un composé racémique de formule :

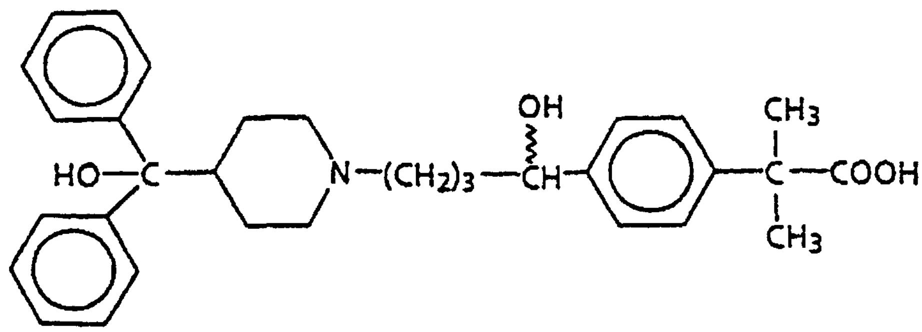

avec une quantité équimolaire d'un agent résolvant optiquement actif, l'acide (+)-di-para-toluyltartrique, dans un solvant organique approprié ;

b) le chauffage de la solution à une température élevée appropriée à la formation d'un sel diastéréoisomère solubilisé entre l'agent résolvant optiquement actif et le composé ;

c) le refroidissement de la solution pendant une durée suffisante pour précipiter le sel diastéréoisomère ;

d) la récupération du sel diastéréoisomère ; et

e) l'hydrolyse du sel diastéréoisomère pour isoler le composé.

**2.** Procédé pour préparer un composé de formule :

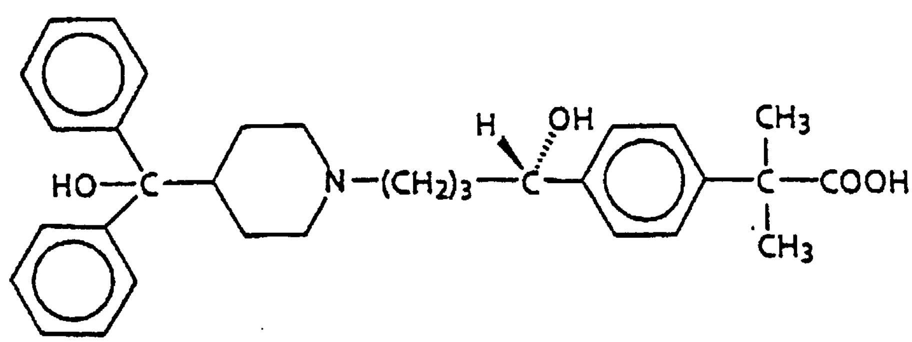

comprenant:

a) la dissolution dans une solution d'une quantité d'un composé racémique de formule :

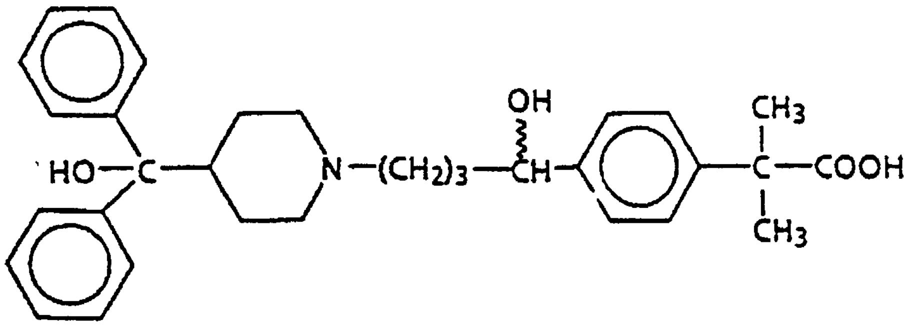

avec une quantité équimolaire d'un agent résolvant optiquement actif, l'acide (-)-di-para-toluyltartrique, dans un solvant organique approprié ;

b) le chauffage de la solution à une température élevée appropriée à la formation d'un sel diastéréoisomère solubilisé entre l'agent résolvant optiquement actif et le composé ;

c) le refroidissement de la solution pendant une durée suffisante pour précipiter le complexe interactif sous forme de sel diastéréoisomère ;

d) la récupération du sel diastéréoisomère ; et

e) l'hydrolyse du sel diastéréoisomère pour isoler le composé.

**3.** Procédé pour préparer un composé de formule :

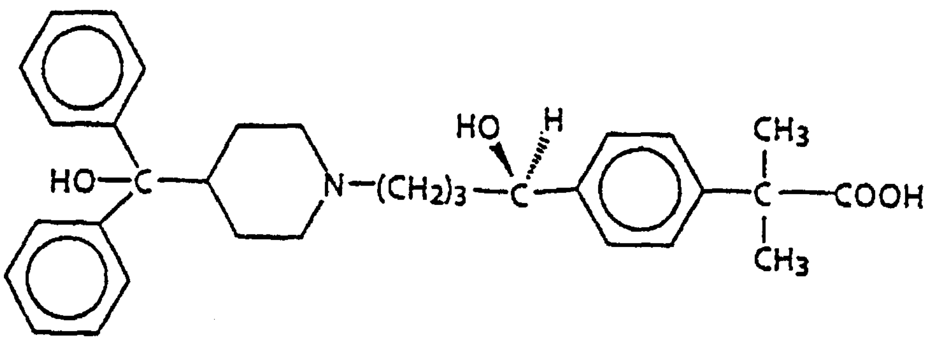

comprenant:

a) la dissolution dans une solution d'une quantité d'un composé racémique de formule :

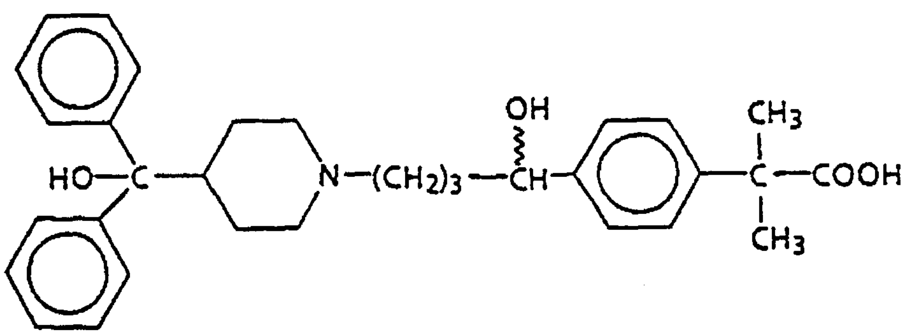

avec une quantité équimolaire d'un agent résolvant optiquement actif, l'acide (-)-di-para-toluyltartrique, dans un solvant organique approprié ;

b) le chauffage de la solution à une température appropriée à la formation d'un premier sel diastéréoisomère solubilisé entre l'agent résolvant optiquement actif et le composé ;

c) le refroidissement de la solution pendant une durée suffisante pour précipiter le premier sel diastéréoisomère ;
d) le retrait du premier sel diastéréoisomère et la conservation de la solution sous forme d'un filtrat ;
e) l'hydrolyse et la séparation du composé du filtrat ;
f) la dissolution en solution du composé avec un agent résolvant optiquement actif, l'acide (+)-di-para-toluyltartrique en une quantité équimolaire à une quantité du composé de manière à former un second sel diastéréoisomère solubilisé entre ceux-ci,
g) la précipitation du second sel diastéréoisomère ;
h) la récupération du second sel diastéréoisomère ; et
i) l'hydrolyse du second sel diastéréoisomère pour isoler le composé.

**4.** Procédé pour préparer un composé de formule :

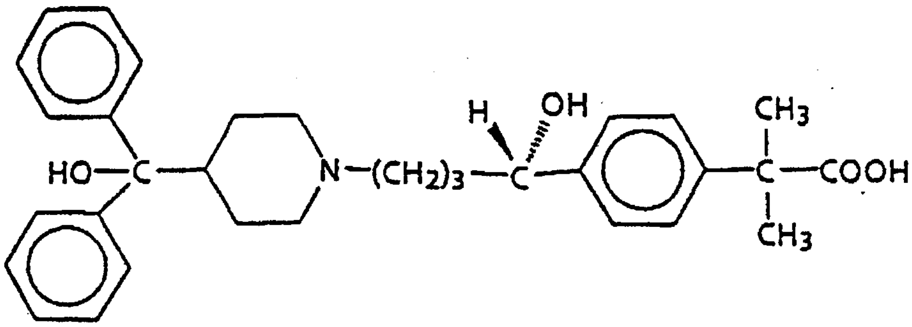

comprenant :

a) la dissolution dans une solution d'une quantité d'un composé racémique de formule :

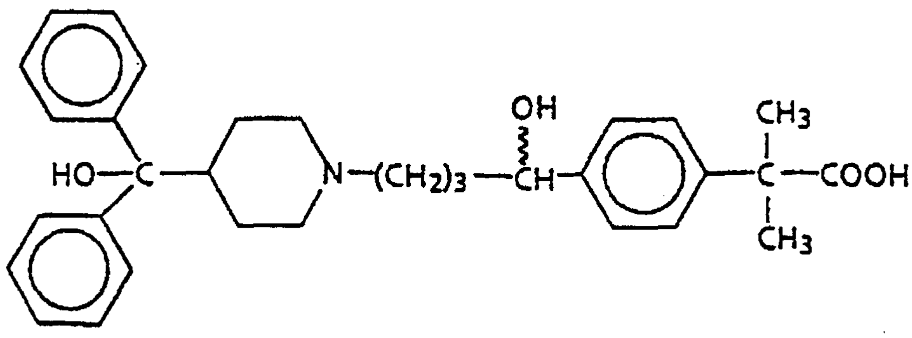

avec une quantité équimolaire d'un agent résolvant optiquement actif, l'acide (+)-di-para-toluyltartrique, dans un solvant organique approprié ;
b) le chauffage de la solution à une température élevée appropriée à la formation d'un premier sel diastéréoisomère solubilisé entre l'agent résolvant optiquement actif et le composé ;
c) le refroidissement de la solution pendant une durée suffisante pour précipiter le premier sel diastéréoisomère ;
d) le retrait du premier sel diastéréoisomère et la conservation de la solution sous forme d'un filtrat ;
e) l'hydrolyse et la séparation du composé du filtrat ;
f) la dissolution en solution du composé avec un agent résolvant optiquement actif, l'acide (-)-di-para-toluyltartrique, en une quantité équimolaire à une quantité du composé de manière à former un second sel diastéréoisomère solubilisé entre ceux-ci ;
g) la précipitation du second sel diastéréoisomère ;
h) la récupération du second sel diastéréoisomère ; et
i) l'hydrolyse du second sel diastéréoisomère pour isoler le composé.

**5.** Composé consistant essentiellement en un sel diastéréoisomère entre l'acide (R)-(+)-4-[4-[4-(hydroxydiphénylméthyl)-1-pipéridinyl]-1-hydroxybutyl]-α,α-diméthylbenzèneacétique et l'acide (2S,3S)-(+)-di-para-toluyltartrique ; ou

composé consistant essentiellement en un sel diastéréoisomère entre l'acide (S)-(-)-4-[4-[4-(hydroxydiphénylméthyl)-1-pipéridinyl]-1-hydroxybutyl]-α,α-diméthylbenzèneacétique et l'acide (2R,3R)-(-)-di-para-toluyl-tartrique.